# EUROPEAN PATENT SPECIFICATION

(11) **EP 3 218 401 B1**
(45) Date of publication and mention of the grant of the patent: **04.03.2020**
(21) Application number: 15802244.2
(22) Date of filing: 09.11.2015
(51) Int. Cl.: C07K 16/22, A61K 39/395, A61K 31/282, A61K 38/21, A61K 45/06, A61K 31/4745, A61K 31/495, A61K 31/513, A61K 31/519, A61K 31/704, A61K 31/7068, A61K 33/24, A61K 31/555, A61K 31/337, A61K 39/00

(54) **PREDICTING RESPONSE TO A VEGF ANTAGONIST**
VORHERSAGE DER REAKTION AUF EINEN VEGF-ANTAGONISTEN
PRÉDICTION DE RÉACTION À UN ANTAGONISTE DU VEGF

(30) Priority: 14.11.2014 US 201462079787 P
(43) Date of publication of application: 20.09.2017
(73) Proprietor: F. Hoffmann-La Roche AG, 4070 Basel (CH)
(72) Inventor: XIAO, Yuanyuan, South San Francisco, CA 94080-4990 (US); BAIS, Carlos, South San Francisco, CA 94080-4990 (US); CHOI, Younjeong, South San Francisco, CA 94080-4990 (US); CONSALVO, Nicola, C., CH-4070 Basel (CH)
(74) Representative: Brodbeck, Michel
(86) International application number: PCT/US2015/059733
(87) International publication number: WO 2016/077227

(56) References cited:
- WO-A1-2011/089101
- WO-A2-2012/118969
- US-A1- 2005 276 808
- US-A1- 2011 206 662
- HAN E S ET AL: "Predictive and prognostic angiogenic markers in a gynecologic oncology group phase II trial of bevacizumab in recurrent and persistent ovarian or peritoneal cancer", GYNECOLOGIC ONCOLOGY, ACADEMIC PRESS, LONDON, GB, vol. 119, no. 3, 1 December 2010 (2010-12-01), pages 484-490, XP027471653, ISSN: 0090-8258, DOI: 10.1016/J.YGYNO.2010.08.016 [retrieved on 2010-09-25]

## Description

### FIELD OF THE INVENTION

The present invention is directed to methods for identifying patients that will benefit from treatment with a VEGF antagonist, e.g., an anti-VEGF antibody.

### BACKGROUND OF THE INVENTION

Angiogenesis is necessary for cancer development, regulating not only primary tumor size and growth, but also impacting invasive and metastatic potential. Accordingly, the mechanisms mediating angiogenic processes have been investigated as potential targets for directed anti-cancer therapies. Early in the study of angiogenic modulators, the vascular endothelial growth factor (VEGF) signaling pathway was discovered to regulate angiogenic activity in multiple cancer types, and multiple therapeutics have been developed to modulate this pathway at various points. Although the use of angiogenesis inhibitors in the clinic has shown success, not all patients respond or fully respond to this therapy. The mechanism(s) underlying such incomplete response is unknown. Therefore, there is a need for the identification of patient subgroups sensitive or responsive to anti-angiogenic cancer therapy.

Bevacizumab (Avastin®) is a recombinant humanized monoclonal IgG1 antibody that specifically binds to and blocks the biological effects of VEGF. Bevacizumab has been approved in Europe for the treatment of the advanced stages of five common types of cancer: colorectal cancer, breast cancer, non-small cell lung cancer (NSCLC), ovarian cancer, and kidney cancer, which collectively cause over 2.5 million deaths each year. In the United States, bevacizumab was the first anti-angiogenesis therapy approved by the FDA, and it is now approved for the treatment of five tumor types: colorectal cancer, NSCLC, brain cancer (glioblastoma), kidney cancer (renal cell carcinoma), and cervical cancer. Over half a million patients have been treated with bevacizumab so far, and a comprehensive clinical program with over 450 clinical trials is investigating the further use of bevacizumab in the treatment of multiple cancer types (including colorectal, breast, NSCLC, brain, gastric, ovarian, and prostate cancers) in different settings (e.g., advanced or early stage disease).

Bevacizumab has shown promise as a co-therapeutic, demonstrating efficacy when combined with a broad range of chemotherapies and other anti-cancer treatments. For example, phase-Ill studies have demonstrated the beneficial effects of combining bevacizumab with standard chemotherapeutic regimens (see, e.g., Saltz et al., 2008, J. Clin. Oncol., 26:2013-2019; Yang et al., 2008, Clin. Cancer Res., 14:5893-5899; Hurwitz et al., 2004, N. Engl. J. Med., 350:2335-2342). However, as in previous studies of angiogenesis inhibitors, some of these phase-Ill studies have shown that a portion of patients experience incomplete response to the addition of bevacizumab to their chemotherapeutic regimens.

Accordingly, there is a need for methods of identifying those patients that are likely to respond or have an improved response to not only angiogenesis inhibitors (e.g., bevacizumab) alone, but also combination therapies comprising angiogenesis inhibitors (e.g., bevacizumab). HAN E S ET AL: "Predictive and prognostic angiogenic markers in a gynecologic oncology group phase II trial of bevacizumab in recurrent and persistent ovarian or peritoneal cancer",GYNECOLOGIC ONCOLOGY, ACADEMIC PRESS, LONDON, GB, vol. 119, no. 3, 1 December 2010 (2010-12-01), pages 484-490,discloses high CD31-MVD as a prognostic marker for Epithelial Ovarian Cancer (EOC).

### SUMMARY OF THE INVENTION

In one aspect, the present invention features a method of treating a patient with a cancer, the method including administering to the patient a therapeutically effective amount of a VEGF antagonist, wherein the patient's cancer has been determined to express CD31 and/or tumor VEGFA at a level more than the median level for CD31 and/or tumor VEGFA expression, respectively, in the cancer type. In a related aspect, the present invention features a VEGF antagonist for use in a method of treating a patient with a cancer, wherein the patient's cancer has been determined to express CD31 and/or tumor VEGFA at a level more than the median level for CD31 and/or tumor VEGFA expression, respectively, in the cancer type, and the method comprises administering to the patient a therapeutically effective amount of the VEGF antagonist.

In a second aspect, the present invention features a method of identifying a patient suffering from a cancer who may benefit from administration of a VEGF antagonist, the method including: a) determining the expression level of CD31 and/or tumor VEGFA in a sample obtained from the patient, wherein expression of CD31 and/or tumor VEGFA at a level more than the median level for CD31 and/or tumor VEGFA expression, respectively, in the cancer type indicates that the patient may benefit from administration of a VEGF antagonist, and optionally b) administering the VEGF antagonist in a therapeutically effective amount to the patient.

In a third aspect, the present invention features a method of predicting responsiveness of a patient to administration of a VEGF antagonist for treatment of a cancer, the method including: a) determining the expression level of CD31 and/or tumor VEGFA in a sample obtained from the patient, wherein expression of CD31 and/or tumor VEGFA at a level more than the median level for CD31 and/or tumor VEGFA expression, respectively, in the cancer type indicates that the patient is more likely to be responsive to the administration of the VEGF antagonist, and optionally b) administering the VEGF antagonist in a therapeutically effective amount to the patient.

In certain embodiments, the sample is a tumor tissue sample. In other embodiments, the sample is obtained before neoadjuvant or adjuvant therapy.

In a further embodiment, the patient's cancer has been determined to express CD31 at a level that is more than the median level for CD31 expression in the cancer type. In particular aspects of this embodiment, the patient's cancer has been determined to express CD31 at a level that is more than the 75^{th} percentile for CD31 expression in the cancer type.

In yet another embodiment, the patient's cancer has been determined to express tumor VEGFA at a level that is more than the median level for tumor VEGFA expression in the cancer type. In particular aspects of this embodiment, the patient's cancer has been determined to express tumor VEGFA at a level that is more than the 75^{th} percentile for tumor VEGFA expression in the cancer type.

In one particular embodiment, the administration of the VEGF antagonist improves progression free survival (PFS) in the patient. In a second particular embodiment, the administration of the VEGF antagonist improves overall survival (OS) in the patient. In a third particular embodiment, the VEGF antagonist is administered in combination with one or more additional chemotherapeutic agents in a chemotherapy regimen.

In some embodiments, the one or more additional chemotherapeutic agents is selected from the group consisting of: a chemotherapeutic agent, HER antibody, antibody directed against a tumor associated antigen, anti-hormonal compound, cardioprotectant, cytokine, EGFR-targeted drug, anti-angiogenic agent, tyrosine kinase, inhibitor, COX inhibitor, non-steroidal anti-inflammatory drug, farnesyl trasferase inhibitor, antibody that binds oncofetal protein CA 125, Her2 vaccine, HER targeting therapy, Raf or ras inhibitor, liposomal doxorubicin, topotecan, taxane, dual tyrosine kinase inhibitor, TLK286, EMD-7200, a medicament that treats nausea, a medicament that prevents or treats skin rash or standard acne therapy, a medicament that treats or prevents diarrhea, a body temperature-reducing medicament, and a hematopoietic growth factor. In some preferred embodiments, the chemotherapeutic agent is gemcitabine, carboplatin, oxaliplatin, irinotecan, fluoropyrimidine (e.g., 5-FU), paclitaxel (e.g., nab-paclitaxel), docetaxel, topotecan, capecitabine, lecovorin, temozolomide, interferon-alpha, or liposomal doxorubicin (e.g., pegylated liposomal doxorubicin).

In certain embodiments, the chemotherapy regimen includes the administration of carboplatin and paclitaxel; carboplatin and gemcitabine; or paclitaxel, topotecan, or pegylated liposomal doxorubicin. In other embodiments, the chemotherapy regimen includes the administration of capecitabine and paclitaxel; or capecitabine and docetaxel. In yet other embodiments, the chemotherapy regimen includes the administration of temozolomide and optionally radiotherapy. In a further embodiment, the chemotherapy regimen includes the administration of fluropyrimidine, irinotecan, cisplatin, fluropyramidine and oxaliplatin; fluropyrimidine and irinotecan; fluropyramidine, lecovorin, and oxaliplatin; or ironotecan, fluoropyrimidine, and leucovorin. In yet a further embodiment, the chemotherapy regimen includes the administration of paclitaxel and topotecan; or paclitaxel and cisplatin. In a final embodiment, the chemotherapy regimen includes the administration of interferon-alpha2a.

In a fourth aspect, the present invention features a method for the prognosis of a patient suffering from cancer, the method including: a) determining the expression level of CD31 in a sample obtained from the patient, b) comparing the expression level of CD31 to the median level for CD31 in the cancer type, and c) determining a prognosis for the patient, wherein a poor prognosis is when expression of CD31 is at a level more than the medial level for CD31 expression.

In certain aspects, the method further includes the step of identifying the patient as likely to benefit from administration of a VEGF antagonist when the patient is determined to have a poor prognosis of survival. In yet another aspect, the method further includes the step of administering a VEGF antagonist in a therapeutically effective amount to the patient, if the patient is determine to have a poor prognosis. In some embodiments, the VEGF antagonist is an anti-VEGF antibody. In preferred embodiments, the anti-VEGF antibody is bevacizumab.

In one embodiment, the method is carried out prior to administering an anti-cancer agent in order to provide a pre-administration prognosis of survival. In a second embodiment, the survival is progression free survival or overall survival.

In a fifth aspect, the present invention features a method of treating a patient with a cancer, the method including administering to the patient a therapeutically effective amount of a therapeutic agent other than a VEGF antagonist, wherein the patient's cancer has been determined to express CD31 and/or tumor VEGFA at a level less than the median level for CD31 and/or tumor VEGFA expression, respectively, in the cancer type. In a related aspect, the present invention features a therapeutic agent other than a VEGF antagonist for use in a method of treating a patient with a cancer, wherein the patient's cancer has been determined to express CD31 and/or tumor VEGFA at a level less than the median level for CD31 and/or tumor VEGFA expression, respectively, in the cancer type, and the method comprises administering to the patient a therapeutically effective amount of the therapeutic agent other than a VEGF antagonist.

In one embodiment, the patient's cancer has been determined to express CD31 at a level that is less than the median level for CD31 expression in the cancer type. In certain aspects of this embodiment, the patient's cancer has been determined to express CD31 at a level that is less than the 25^{th} percentile for CD31 expression in the cancer type.

In another embodiment, the patient's cancer has been determined to express tumor VEGFA at a level that is less than the median level for tumor VEGFA expression in the cancer type. In certain aspects of this embodiment, the patient's cancer has been determined to express tumor VEGFA at a level that is less than the 25^{th} percentile for tumor VEGFA expression in the cancer type.

In particular embodiments of the present inventions, the cancer is selected from the group consisting of colorectal cancer, breast cancer, non-small cell lung cancer (NSCLC), kidney cancer (renal cell carcinoma), or brain cancer (glioblastoma). In other particular embodiments of the present inventions, the cancer is a gynecologic cancer selected from the group consisting of ovarian cancer, peritoneal cancer, fallopian tube cancer, cervical cancer, endometrial cancer, vaginal cancer, and vulvar cancer. In preferred embodiments, the gynecologic cancer is ovarian cancer. In yet another embodiment of the present inventions, the cancer is platinum-resistant, platinum-sensitive, advanced, refractory, or recurrent.

In a further embodiment, the level of CD31 expression detected in the sample of the patient is used to determine the density of CD31 microvascular structures (CD31 MVD) in the cancer of the patient, and optionally wherein CD31 MVD of the patient's sample is compared to the median level of CD31 MVD in the cancer type. In some particular embodiments, CD31 and/or tumor VEGFA expression is detected by an immunohistochemical (IHC) method.

In some preferred embodiments, the VEGF antagonist is an anti-VEGF antibody. In particular preferred embodiments, the anti-VEGF antibody is bevacizumab.

### BRIEF DESCRIPTION OF THE DRAWINGS

**FIG. 1** is a schematic showing a phase III trial design for assessing the effects of adding bevacizumab to a carboplatin-paclitaxel chemotherapy regimen for the treatment of stage III/IV ovarian cancer.
**FIG. 2** is a graph showing the distribution of CD31 expression in a sample patient population.
**FIG. 3** is a table showing the results of a statistical analysis and a Forest plot of progression free survival (PFS) for control (paclitaxel-carboplatin-placebo (CPP)) vs. bevacizumab maintenance (paclitaxel-carboplatin-bevacizumab (CPB15+)) according to tumor cell biomarker subgroups at specified cutoffs.
**FIG. 4** shows the Kaplan Meier curves of PFS for CD31 high and low expressors (dichotomized at 50% cutoff) comparing CPP vs. CPB15+.
**FIG. 5** is a table showing the results of a statistical analysis and a Forest plot of PFS for CPP vs. CPB15+ according to CD31 subgroups dichotomized at 25%, 50%, and 75% cutoffs for CD31 expression.
**FIG. 6** is a table showing hazard ratio (HR) estimates for PFS treatment effects within the indicated CD31 subgroups, adjusting for patient performance status, disease stage, and debulking surgery outcome.
**FIG. 7** is a table showing the results of a statistical analysis and a Forest plot of overall survival (OS) for CPP vs. CPB15+ according to tumor cell biomarker subgroups at specific cutoffs.
**FIG. 8** is a table showing the results of a statistical analysis and a Forest plot of OS for CPP vs. CPB15+ according to CD31 subgroups dichotomized at 25%, 50%, and 75% cutoff for CD31 expression.
**FIG. 9A** shows the Kaplan Meier curve of OS for CD31 high and low expressors (dichotomized at 50% cutoff), comparing CPP vs. CPB15+.
**FIG. 9B** shows the Kaplan Meier curve of OS for CD31 high and low expressors (dichotomized at 75% cutoff), comparing CPP vs. CPB15+.
**FIG. 10** is a table showing HR estimates for OS treatment effects within the indicated subgroups, adjusting for patient performance status, disease stage, and debulking surgery outcome.
**FIG. 11** is subpopulation treatment effect pattern plot (STEPP) analysis comparing PFS (left graph) and OS (right graph) between the treatment arms (CPP vs. CPB15+) within predefined patient subgroups based on CD31 levels. Each dot on the middle, solid curve represents 25% of the population.
**FIG. 12** shows the Kaplan Meier curves of PFS for tumor VEGFA high and low expressors (dichotomized at 75% cutoff) comparing CPP vs. CPB15+.
**FIG. 13** shows the Kaplan Meier curves of OS for tumor VEGFA high and low expressors (dichotomized at 75% cutoff) comparing CPP vs. CPB15+.
**FIG. 14** is a table showing HR estimates for PFS and OS treatment effects within the indicated treatment groups, adjusting for patient performance status, disease stage, and debulking surgery outcome.
**FIG. 15** is a table showing the results of a statistical analysis and a Forest plot of PFS for CPP vs. CPB15+ according to VEGF subgroups dichotomized at 25%, 50%, and 75% cutoffs for tumor VEGFA expression.
**FIG. 16** is a table showing the results of a statistical analysis and a Forest plot of OS for CPP vs. CPB15+ according to tumor VEGFA subgroups dichotomized at 25%, 50%, and 75% cutoffs for tumor VEGFA expression.
**FIG. 17** is subpopulation treatment effect pattern plot (STEPP) analysis comparing PFS (left graph) and OS (right graph) between the treatment arms (CPP vs. CPB15+) within predefined patient subgroups based on tumor VEGFA levels. Each dot on the middle, solid curve represents 25% of the population.

### DETAILED DESCRIPTION OF THE INVENTION

### I. Introduction

The present invention is based in part on the finding that the tumor expression levels of CD31 and/or tumor VEGFA in a given patient, relative to the expression levels in a given population of patients having a cancer, in particular, a gynecologic cancer, such as ovarian cancer, associate with treatment effects in those patients administered an angiogenesis inhibitor in combination with a chemotherapy regimen. Specifically, variations in higher microvasculature density levels (as measured by the number of CD31 vascular structures per mm²) and/or tumor VEGFA were identified as candidate markers/predictors for improved progression-free survival (PFS) and improved overall survival (OS) of ovarian cancer patients in response to the addition of bevacizumab to carboplatin-paclitaxel chemotherapeutic regimens. Patients exhibiting a response or sensitivity to the addition of bevacizumab to these chemotherapy regimens were identified to have increased expression of CD31 and/or tumor VEGFA relative to expression levels in a given population of patients diagnosed with or having a cancer, in particular, a gynecologic cancer, such as ovarian cancer. In accordance with the present invention, it was discovered that a greater bevacizumab treatment effect was associated with high CD31 microvascular density (CD31 MVD) expression and/or high tumor VEGFA expression in tumor cells.

The invention thus provides methods of treating patients with cancer (e.g., a gynecologic cancer (e.g., ovarian, peritoneal, fallopian tube, cervical, endometrial, vaginal, or vulvar cancer) or breast cancer (e.g., metastatic breast cancer (MBC); also see below)) by administering to the patients a VEGF antagonist (e.g., an anti-VEGF antibody, such as bevacizumab), where the patient's cancer has been determined to express CD31 MVD and/or tumor VEGFA at a level higher than the median level in the cancer type. The invention also provides methods of identifying patients with cancer (e.g., a gynecologic cancer (e.g., ovarian, peritoneal, fallopian tube, cervical, endometrial, vaginal, or vulvar cancer) or breast cancer (e.g., metastatic breast cancer (MBC); also see below)) who may benefit from administration of a VEGF antagonist (e.g., an anti-VEGF antibody, such as bevacizumab), optionally in addition to another anti-cancer therapy, or identifying a patient who may be more responsive to treatment with a VEGF antagonist (e.g., an anti-VEGF antibody, such as bevacizumab) by determining expression level of CD31 and/or tumor VEGFA in a tumor sample from the patient, where the VEGF antagonist (e.g., an anti-VEGF antibody, such as bevacizumab) is administered if the expression of CD31 MVD and/or tumor VEGFA is at a level more than the median level for CD31 MVD and/or tumor VEGFA expression in the cancer type.

### II. Definitions

Protein "expression" refers to conversion of the information encoded in a gene into messenger RNA (mRNA) and then to the protein.

A sample or cell that "expresses" a protein of interest (such as CD31 and/or tumor VEGFA) is one in which mRNA encoding the protein, or the protein, including fragments thereof, is determined to be present in the sample or cell.

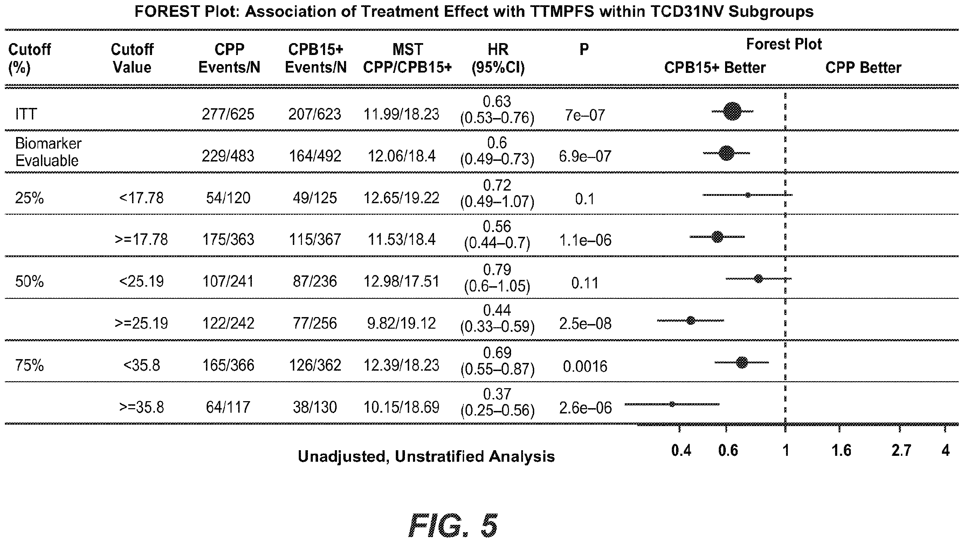

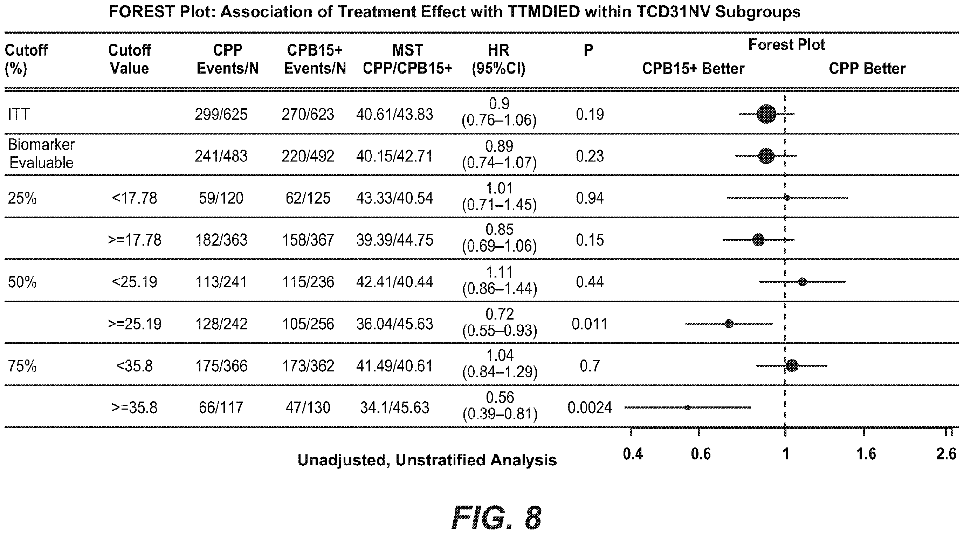

A sample, cell, tumor, or cancer which "has been determined to express" or "expresses" CD31 MVD and/or tumor VEGFA at a level more than the median level for CD31 MVD and/or tumor VEGFA expression" in a type of cancer is one in which the level of CD31 MVD and/or tumor VEGFA expression is considered a "high CD31 MVD and/or tumor VEGFA level" to a skilled person for that type of cancer. Generally, such level will be in the range from about 50% up to about 100% (e.g., 50%, 55%, 60%, 65%, 70%, 75%, 80%, 85%, 90%, or 95%) relative to CD31 MVD and/or tumor VEGFA levels in a population of samples, cells, tumors, or cancers of the same cancer type. For instance the population that is used to arrive at the median expression level may be ovarian cancer samples generally, or subgroupings thereof, such as chemotherapy-resistant ovarian cancer, platinum-resistant ovarian cancer, as well as advanced, refractory, or recurrent ovarian cancer. The examples herein demonstrate how the median expression level can be determined. This may constitute an absolute value of expression. Thus, with reference to Figs. 5 and 8 herein, the cutoff for ovarian patients considered to express CD31 MVD at a high level may be about 17.78 or more (25^{th} percentile), about 25.19 or more (50^{th} percentile), about 35.8 or more (75^{th} percentile), etc. Such absolute values will be quantified in an assay under specified assay conditions, such as in an immunohistochemical (IHC) method, e.g., as disclosed herein, and most preferably the IHC assay as in Example 1. Preferably, the level of CD31 MVD and/or tumor VEGFA expression is more than or at the 50^{th} percentile (e.g., the 50^{th}, 55^{th}, 60^{th}, 65^{th}, 68^{th}, or 70^{th} percentile), and most preferably more than or at the 75^{th} percentile (e.g., the 75^{th}, 76^{th}, 78^{th}, 80^{th}, 85^{th}, 90^{th}, or 95^{th} percentile). By "cancer is or has been determined to express" or "cancer expresses," used in reference to a particular biomarker (e.g., CD31 and/or tumor VEGFA), means expression of the biomarker (e.g., CD31 and/or tumor VEGFA), as determined using a diagnostic test, any of the detection methods described herein, or the similar. In the case of CD31, expression is in endothelial cells of blood vessels within a cancer or tumor tissue, while VEGFA expression occurs in cancer or tumor cells. Further with respect to CD31, in the methods of the invention, the level of CD31 expression detected in a patient sample can be used to determine the density of CD31 microvascular structures (CD31 MVD) in the cancer of the patient, and optionally the CD31 MVD of the patient's sample can be compared to the median level of CD31 MVD in the cancer type.

By "tissue or cell sample" is meant a collection of cells obtained from a tissue of a subject or patient. The source of the tissue or cell sample may be solid tissue as from a fresh, frozen, and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject or plasma. The tissue sample may also be primary or cultured cells or cell lines. Optionally, the tissue or cell sample is obtained from a cancerous tissue/organ. The tissue sample may contain compounds which are not naturally intermixed with the tissue in nature such as preservatives, anticoagulants, buffers, fixatives, nutrients, antibiotics, or the like. For the purposes herein a "section" of a tissue sample is meant a single part or piece of a tissue sample, e.g. a thin slice of tissue or cells cut from a tissue sample.

A "tumor sample" herein is a sample derived from, or comprising tumor cells from, a patient's tumor. Examples of tumor samples herein include, but are not limited to, tumor biopsies, circulating tumor cells, circulating plasma proteins, ascitic fluid, primary cell cultures or cell lines derived from tumors or exhibiting tumor-like properties, as well as preserved tumor samples, such as formalin-fixed, paraffin-embedded tumor samples or frozen tumor samples. In addition to tumor cells, tumor samples can include blood vessels.

By "associate" or "association" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

The term "biomarker" as used herein refers generally to a molecule, including a gene, protein, carbohydrate structure, or glycolipid, the expression of which in or on a mammalian tissue or cell can be detected by standard methods (or methods disclosed herein) and is predictive, diagnostic, and/or prognostic for the sensitivity of a mammalian cell or tissue to treatment regimes based on inhibition of angiogenesis using, e.g., an anti-angiogenesis agent such as a VEGF-specific inhibitor (e.g., an anti-VEGF antibody, such as bevicizumab). Optionally, the expression of such a biomarker is determined to be higher than that observed for a control/reference tissue or cell sample. Expression of such biomarkers can be determined using a high-throughput multiplexed immunoassay such as those commercially available from Rules Based Medicine, Inc. or Meso Scale Discovery. Expression of the biomarkers may also be determined using, e.g., PCR or FACS assay, an immunohistochemical assay or a gene chip-based assay.

A "VEGF antagonist" or "VEGF-specific antagonist" refers to a molecule capable of binding to VEGF, reducing VEGF expression levels, or neutralizing, blocking, inhibiting, abrogating, reducing, or interfering with VEGF biological activities, including, but not limited to, VEGF binding to one or more VEGF receptors, VEGF signaling, and VEGF mediated angiogenesis and endothelial cell survival or proliferation. For example, a molecule capable of neutralizing, blocking, inhibiting, abrogating, reducing, or interfering with VEGF biological activities can exert its effects by binding to one or more VEGF receptor (VEGFR) (e.g., VEGFR1, VEGFR2, VEGFR3, membrane-bound VEGF receptor (mbVEGFR), or soluble VEGF receptor (sVEGFR)). Included as VEGF-specific antagonists useful in the methods of the invention are polypeptides that specifically bind to VEGF, anti-VEGF antibodies and antigen-binding fragments thereof, receptor molecules and derivatives which bind specifically to VEGF thereby sequestering its binding to one or more receptors, fusions proteins (e.g., VEGF-Trap (Regeneron)), and VEGF₁₂₁-gelonin (Peregrine). VEGF-specific antagonists also include antagonist variants of VEGF polypeptides, antisense nucleobase oligomers complementary to at least a fragment of a nucleic acid molecule encoding a VEGF polypeptide; small RNAs complementary to at least a fragment of a nucleic acid molecule encoding a VEGF polypeptide; ribozymes that target VEGF; peptibodies to VEGF; and VEGF aptamers. VEGF antagonists also include polypeptides that bind to VEGFR, anti-VEGFR antibodies, and antigen-binding fragments thereof, and derivatives which bind to VEGFR thereby blocking, inhibiting, abrogating, reducing, or interfering with VEGF biological activities (e.g., VEGF signaling), or fusions proteins. VEGF-specific antagonists also include nonpeptide small molecules that bind to VEGF or VEGFR and are capable of blocking, inhibiting, abrogating, reducing, or interfering with VEGF biological activities. Thus, the term "VEGF activities" specifically includes VEGF mediated biological activities of VEGF. In certain embodiments, the VEGF antagonist reduces or inhibits, by at least 10%, 20%, 30%, 40%, 50%, 60%, 70%, 80%, 90% or more, the expression level or biological activity of VEGF. In some embodiments, the VEGF inhibited by the VEGF-specific antagonist is VEGF (8-109), VEGF (1-109), or VEGF₁₆₅.

As used herein VEGF antagonists can include, but are not limited to, anti-VEGFR2 antibodies and related molecules (e.g., ramucirumab, tanibirumab, aflibercept), anti-VEGFR1 antibodies and related molecules (e.g., icrucumab, aflibercept (VEGF Trap-Eye; EYLEA®), and ziv-aflibercept (VEGF Trap; ZALTRAP®)), bispecific VEGF antibodies (e.g., MP-0250, vanucizumab (VEGF-ANG2), and bispecific antibodies disclosed in US 2001/0236388), bispecific antibodies including combinations of two of anti-VEGF, anti-VEGFRl, and anti-VEGFR2 arms, anti-VEGFA antibodies (e.g., bevacizumab, sevacizumab), anti-VEGFB antibodies, anti-VEGFC antibodies (e.g., VGX-100), anti-VEGFD antibodies, and nonpeptide small molecule VEGF antagonists (e.g., pazopanib, axitinib, vandetanib, stivarga, cabozantinib, lenvatinib, nintedanib, orantinib, telatinib, dovitinig, cediranib, motesanib, sulfatinib, apatinib, foretinib, famitinib, and tivozanib).

An "anti-VEGF antibody" is an antibody that binds to VEGF with sufficient affinity and specificity. In certain embodiments, the antibody will have a sufficiently high binding affinity for VEGF, for example, the antibody may bind hVEGF with a K_{d} value of between 100 nM-1 pM. Antibody affinities may be determined, e.g., by a surface plasmon resonance based assay (such as the BIAcore assay as described in PCT Application Publication No. WO2005/012359); enzyme-linked immunoabsorbent assay (ELISA); and competition assays (e.g. RIA's).

In certain embodiments, the anti-VEGF antibody can be used as a therapeutic agent in targeting and interfering with diseases or conditions wherein the VEGF activity is involved. Also, the antibody may be subjected to other biological activity assays, e.g., in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (U.S. Pat. No. 5,500,362); and agonistic activity or hematopoiesis assays (see WO 95/27062). An anti-VEGF antibody will usually not bind to other VEGF homologues such as VEGF-B or VEGF-C, nor other growth factors such as P1GF, PDGF, or bFGF. In one embodiment, anti-VEGF antibody is a monoclonal antibody that binds to the same epitope as the monoclonal anti-VEGF antibody A4.6.1 produced by hybridoma ATCC HB 10709. In another embodiment, the anti-VEGF antibody is a recombinant humanized anti-VEGF monoclonal antibody generated according to Presta et al. (1997) Cancer Res. 57:4593-4599, including but not limited to the antibody known as bevacizumab (BV; AVASTIN®).

The anti-VEGF antibody "Bevacizumab (BV)," also known as "rhuMAb VEGF" or "AVASTIN®," is a recombinant humanized anti-VEGF monoclonal antibody generated according to Presta et al. (1997) Cancer Res. 57:4593-4599. It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and other humanized anti-VEGF antibodies are further described in U.S. Pat. No. 6,884,879 issued Feb. 26, 2005. Additional preferred antibodies include the G6 or B20 series antibodies (e.g., G6-31, B20-4.1), as described in PCT Application Publication No. WO 2005/012359. For additional preferred antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., Journal of Immunological Methods 288:149-164 (2004). Other preferred antibodies include those that bind to a functional epitope on human VEGF comprising of residues F17, M18, D19, Y21, Y25, Q89, 191, K101, E103, and C104 or, alternatively, comprising residues F17, Y21, Q22, Y25, D63, 183, and Q89.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

A "blocking" antibody or an antibody "antagonist" is one which inhibits or reduces biological activity of the antigen it binds. For example, a VEGF-specific antagonist antibody binds VEGF and inhibits the ability of VEGF to induce vascular endothelial cell proliferation. Preferred blocking antibodies or antagonist antibodies completely inhibit the biological activity of the antigen.

Unless indicated otherwise, the expression "multivalent antibody" is used throughout this specification to denote an antibody comprising three or more antigen binding sites. The multivalent antibody is preferably engineered to have the three or more antigen binding sites and is generally not a native sequence IgM or IgA antibody.

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the V_{H}-V_{L} dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

"Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. Proc. Natl. Acad. Sci. 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol., 147 (1):86-95 (1991); and U.S. Pat. No. 5,750,373.

An "isolated" polypeptide or "isolated" antibody is one that has been identified and separated and/or recovered from a component of its natural environment. Contaminant components of its natural environment are materials that would interfere with diagnostic or therapeutic uses for the polypeptide or antibody, and may include enzymes, hormones, and other proteinaceous or nonproteinaceous solutes. In preferred embodiments, the polypeptide or antibody will be purified (1) to greater than 95% by weight of polypeptide or antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated polypeptide or antibody includes the polypeptide or antibody in situ within recombinant cells since at least one component of the polypeptide's natural environment will not be present. Ordinarily, however, isolated polypeptide or antibody will be prepared by at least one purification step.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, methods and compositions of the invention are useful in attempts to delay development of a disease or disorder.

The terms "therapeutically effective amount" or "effective amount" refer to an amount of a drug effective to treat cancer in the patient. The effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. The effective amount may extend progression free survival (e.g. as measured by Response Evaluation Criteria for Solid Tumors, RECIST, or CA-125 changes), result in an objective response (including a partial response, PR, or complete response, CR), improve survival (including overall survival and progression free survival) and/or improve one or more symptoms of cancer (e.g. as assessed by FOSI). Most preferably, the therapeutically effective amount of the drug is effective to improve progression free survival (PFS) and/or overall survival (OS).

"Survival" refers to the patient remaining alive, and includes overall survival as well as progression free survival.

"Overall survival" refers to the patient remaining alive for a defined period of time, such as 1 year, 5 years, etc. from the time of diagnosis or treatment.

The phrase "progression-free survival" in the context of the present invention refers to the length of time during and after treatment during which, according to the assessment of the treating physician or investigator, a patient's disease does not become worse, i.e., does not progress. As the skilled person will appreciate, a patient's progression-free survival is improved or enhanced if the patient experiences a longer length of time during which the disease does not progress as compared to the average or mean progression free survival time of a control group of similarly situated patients.

By "extending survival" is meant increasing overall or progression free survival in a treated patient relative to an untreated patient (i.e., relative to a patient not treated with a VEGF antagonist (e.g., an anti-VEGF antibody, such as bevacizumab), or relative to a patient who does not express CD31 or tumor VEGFA at the designated level, and/or relative to a patient treated with an approved anti-tumor agent (such as topotecan or liposomal doxorubicin, where the cancer is ovarian cancer).

A "fixed" or "flat" dose of a therapeutic agent herein refers to a dose that is administered to a human patient without regard for the weight (WT) or body surface area (BSA) of the patient. The fixed or flat dose is therefore not provided as a mg/kg dose or a mg/m² dose, but rather as an absolute amount of the therapeutic agent.

A "loading" dose herein generally comprises an initial dose of a therapeutic agent administered to a patient, and is followed by one or more maintenance dose(s) thereof. Generally, a single loading dose is administered, but multiple loading doses are contemplated herein. Usually, the amount of loading dose(s) administered exceeds the amount of the maintenance dose(s) administered and/or the loading dose(s) are administered more frequently than the maintenance dose(s), so as to achieve the desired steady-state concentration of the therapeutic agent earlier than can be achieved with the maintenance dose(s).

A "maintenance" dose or "extended" dose herein refers to one or more doses of a therapeutic agent administered to the patient over a treatment period. Usually, the maintenance doses are administered at spaced treatment intervals, such as approximately every week, approximately every 2 weeks, approximately every 3 weeks, or approximately every 4 weeks.

The phrase "responsive to" in the context of the present invention indicates that a subject/patient suffering, suspected to suffer or prone to suffer from cancer (e.g., a gynecologic cancer (e.g., ovarian, peritoneal, fallopian tube, cervical, endometrial, vaginal, or vulvar cancer) or breast cancer (e.g., MBC; also see below)), shows a response to a chemotherapy regimen comprising the addition of an anti-VEGF agent, such as an anti-VEGF antibody, e.g., bevacizumab. A skilled person will readily be in a position to determine whether a person treated with an anti-VEGF agent, such as an anti-VEGF antibody, e.g., bevacizumab according to the methods of the invention shows a response. For example, a response may be reflected by decreased suffering from ovarian cancer, such as a diminished and/or halted tumor growth, reduction of the size of a tumor, and/or amelioration of one or more symptoms of ovarian cancer, e.g., ovarian bleeding, pain, anemia. Preferably, the response may be reflected by decreased or diminished indices of the metastatic conversion of the cancer or indices of the cancer, e.g., the prevention of the formation of metastases or a reduction of number or size of metastases.

The phrase "a patient suffering from" in accordance with the invention refers to a patient showing clinical signs of cancer (e.g., a gynecologic cancer (e.g., ovarian, peritoneal, fallopian tube, cervical, endometrial, vaginal, or vulvar cancer) or breast cancer (e.g., metastatic MBC; also see below)). The phrase "being susceptible to" or "being prone to," in the context of cancer, refers to an indication disease in a patient based on, e.g., a possible genetic predisposition, a pre- or eventual exposure to hazardous and/or carcinogenic compounds, or exposure to carcinogenic physical hazards, such as radiation.

The terms "administration" or "administering" as used herein mean the administration of an angiogenesis inhibitor, e.g., an anti-VEGF antibody, such as bevacizumab, and/or a pharmaceutical composition/treatment regimen comprising an angiogenesis inhibitor, e.g., an anti-VEGF antibody, such as bevacizumab, to a patient in need of such treatment or medical intervention by any suitable means known in the art for administration of a therapeutic antibody. Nonlimiting routes of administration include by oral, intravenous, intraperitoneal, subcutaneous, intramuscular, topical, intradermal, intranasal or intrabronchial administration (for example as effected by inhalation). Particularly preferred in context of this invention is parenteral administration, e.g., intravenous administration. With respect to bevacizumab for the treatment of colorectal cancer, the preferred dosages according to the EMEA are 5 mg/kg or 10 mg/kg of body weight given once every 2 weeks or 7.5 mg/kg or 15 mg/kg of body weight given once every 3 weeks. For the treatment of NSCLC, the preferred dosage is 15 mg/kg given once every 3 weeks by infusion in combination with carboplatin and paclitaxel. For the treatment of renal cell carcinoma, the preferred dosage is 10 mg/kg given once every 2 weeks by infusion with interferon α-2a or as a monotherapy. For the treatment of cervical cancer, the preferred dosage is 15 mg/kg given once every three weeks by infusion and administered in combination with one of the following chemotherapy regimens: paclitaxel and cisplatin or paclitaxel and topotecan. For the treatment of glioblastoma, the preferred dosage is 10 mg/kg given once every two weeks by infusion.

The terms "cancer" and "cancerous" refer to or describe the physiological condition in mammals that is typically characterized by unregulated cell growth. Included in this definition are benign and malignant cancers. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

A "cancer type which is able to respond to a VEGF antagonist" is one which when treated with a VEGF antagonist, such as a VEGF antibody (e.g., bevacizumab) or small molecule inhibitor, shows a therapeutically effective benefit in the patient therewith according to any of the criteria for therapeutic effectiveness known to the skilled oncologist, including those elaborated herein, but particularly in terms of survival, including progression free survival (PFS) and/or overall survival (OS). Preferably, such cancer is selected from a gynecologic cancer (e.g., ovarian cancer, peritoneal cancer, fallopian tube cancer, cervical cancer, endometrial cancer, vaginal cancer, and vulvar cancer), breast cancer (e.g., MBC), non-small cell lung cancer (NSCLC), prostate cancer, and colorectal cancer. Most preferably, the cancer is a gynecologic cancer (e.g., ovarian, peritoneal, fallopian tube, cervical, endometrial, vaginal, and vulvar cancer, including platinum-resistant forms of such cancers) or breast cancer, and/or advanced, refractory, or recurrent forms thereof.

An "advanced" cancer is one which has spread outside the site or organ of origin, either by local invasion or metastasis.

A "refractory" cancer is one which progresses even though an anti-tumor agent, such as a chemotherapeutic agent, is being administered to the cancer patient. An example of a refractory cancer is one which is platinum refractory.

A "recurrent" cancer is one which has regrown, either at the initial site or at a distant site, after a response to initial therapy.

Herein, a "patient" is a human patient. The patient may be a "cancer patient," i.e., one who is suffering or at risk for suffering from one or more symptoms of cancer.

Where a VEGF antagonist is administered as a "single anti-tumor agent" it is the only anti-tumor agent administered to treat the cancer, i.e., it is not administered in combination with another anti-tumor agent, such as chemotherapy.

By "standard of care" herein is intended the anti-tumor agent or agents that are routinely used to treat a particular form of cancer. For example, for platinum-resistant ovarian cancer, astandard of care is topotecan or liposomal doxorubicin.

A "chemotherapeutic agent" includes chemical compounds useful in the treatment of cancer. Examples of chemotherapeutic agents include erlotinib (TARCEVA®, Genentech/OSI Pharm.), bortezomib (VELCADE®, Millennium Pharm.), disulfiram, epigallocatechin gallate , salinosporamide A, carfilzomib, 17-AAG (geldanamycin), radicicol, lactate dehydrogenase A (LDH-A), fulvestrant (FASLODEX®, AstraZeneca), sunitib (SUTENT®, Pfizer/Sugen), letrozole (FEMARA®, Novartis), imatinib mesylate (GLEEVEC®, Novartis), finasunate (VATALANIB®, Novartis), oxaliplatin (ELOXATIN®, Sanofi), 5-FU (5-fluorouracil), leucovorin, Rapamycin (Sirolimus, RAPAMUNE®, Wyeth), Lapatinib (TYKERB®, GSK572016, Glaxo Smith Kline), Lonafamib (SCH 66336), sorafenib (NEXAVAR®, Bayer Labs), gefitinib (IRESSA®, AstraZeneca), AG1478, alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin 1 and cryptophycin 8); adrenocorticosteroids (including prednisone and prednisolone); cyproterone acetate; 5α-reductases including finasteride and dutasteride); vorinostat, romidepsin, panobinostat, valproic acid, mocetinostat dolastatin; aldesleukin, talc duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin γ1I and calicheamicin ω1I (Angew Chem. Intl. Ed. Engl. 1994 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL (paclitaxel; Bristol-Myers Squibb Oncology, Princeton, N.J.), ABRAXANE® (Cremophor-free), albumin-engineered nanoparticle formulations of paclitaxel (American Pharmaceutical Partners, Schaumberg, Ill.), and TAXOTERE® (docetaxel, doxetaxel; Sanofi-Aventis); chloranmbucil; GEMZAR® (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, iodoxyfene , 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) antiandrogens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN®, rIL-2; a topoisomerase 1 inhibitor such as LURTOTECAN®; ABARELIX® rmRH; and (ix) pharmaceutically acceptable salts, acids and derivatives of any of the above.

Chemotherapeutic agent also includes antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full-length IgG1 λ antibody genetically modified to recognize interleukin-12 p40 protein.

Chemotherapeutic agent also includes "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6. 3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA®) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-1 inhibitors such as antisense agent ISIS-5132 available from ISIS Pharmaceuticals which inhibit Raf-1 signaling; non-HER targeted TK inhibitors such as imatinib mesylate (GLEEVEC®, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis(4-fluoroanilino)phthalimide); tyrphostines containing nitrothiophene moieties; PD-0183805 (Warner-Lamber); antisense molecules (e.g. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVEC®); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE®); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

By "platinum-based chemotherapeutic agent" or "platin" is meant an antineoplastic drug that is a coordination complex of platinum. Examples of platinum-based chemotherapeutic agents include carboplatin, cisplatin, and oxaliplatinum.

By "platinum-based chemotherapy" is meant therapy with one or more platinum-based chemotherapeutic agent, optionally in combination with one or more other chemotherapeutic agents.

By "chemotherapy-resistant" cancer is meant cancer in a patient that has progressed while the patient is receiving a chemotherapy regimen (i.e., the patient is "chemotherapy refractory"), or the patient has progressed within 12 months (for instance, within 6 months) after completing a chemotherapy regimen.

By "platinum-resistant" cancer is meant cancer in a patient that has progressed while receiving platinum-based chemotherapy (i.e., the patient is "platinum refractory"), or the patient has progressed within 12 months (for instance, within 6 months) after completing a platinum-based chemotherapy regimen.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

The term "benefit" is used in the broadest sense and refers to any desirable effect and specifically includes clinical benefit as defined herein. Clinical benefit can be measured by assessing various endpoints, e.g., inhibition, to some extent, of disease progression, including slowing down and complete arrest; reduction in the number of disease episodes and/or symptoms; reduction in lesion size; inhibition (i.e., reduction, slowing down or complete stopping) of disease cell infiltration into adjacent peripheral organs and/or tissues; inhibition (i.e. reduction, slowing down or complete stopping) of disease spread; decrease of auto-immune response, which may, but does not have to, result in the regression or ablation of the disease lesion; relief, to some extent, of one or more symptoms associated with the disorder; increase in the length of disease-free presentation following treatment, e.g., progression-free survival; increased overall survival; higher response rate; and/or decreased mortality at a given point of time following treatment.

### III. Methods

### A. Methods of Treatment

The invention herein provides methods for treating patients with a type of cancer that is able to respond to a VEGF antagonist (e.g., an anti-VEGF antibody, such as bevacizumab), involving administering a therapeutically effective amount of the antagonist to the patient, wherein the patient's cancer has been determined to express CD31 MVD and/or tumor VEGFA at a level more than the median level for CD31 MVD and/or tumor VEGFA expression in the cancer type. Preferably the patient's cancer has been determined to express CD31 MVD and/or tumor VEGFA at a level which is more than the 50^{th} percentile, most preferably greater than the 75^{th} percentile for CD31 and/or tumor VEGFA expression in the cancer type.

In a particular embodiment, the invention provides methods for treating patients with a gynecologic cancer (e.g., ovarian, peritoneal, fallopian tube, cervical, endometrial, vaginal, or vulvar cancer) or breast cancer (e.g., MBC), involving administering a therapeutically effective amount of a VEGF antagonist (e.g., an anti-VEGF antibody, such as bevacizumab) to the patient, wherein the patient's cancer has been determined to express CD31 MVD at a level more than the median level for CD31 MVD expression in the cancer, and/or wherein the patient's cancer sample has been determined to express CD31 MVD at a level which is greater than the 75^{th} percentile for CD31 expression the cancer, and/or expresses tumor VEGFA at a level that is greater than the median level, and/or wherein the patient's cancer sample has been determined to express tumor VEGFA at a level which is greater than the 75^{th} percentile for tumor VEGFA expression in the cancer. Optionally, these methods involve the co-administration of the VEGF antagonist with one or more additional chemotherapeutic agents (e.g., carboplatin and/or paclitaxel), as described further below.

In another aspect, the invention provides methods for selecting a therapy for patients with a type of cancer that is able to respond to the administration of a VEGF antagonist (e.g., an anti-VEGF antibody, such as bevacizumab), optionally in combination with one or more additional chemotherapeutic agents, involving determining CD31 MVD and/or tumor VEGFA expression in a cancer sample from the patient and selecting a VEGF antagonist (e.g., an anti-VEGF antibody, such as bevacizumab) if the cancer sample has been determined to have CD31 MVD and/or express tumor VEGFA at a level greater than the median level for CD31 MVD and/or tumor VEGFA expression in the cancer type. In this embodiment, preferably the cancer type is a gynecologic cancer (e.g., ovarian, peritoneal, fallopian tube, cervical, endometrial, vaginal, or vulvar cancer) or breast cancer (e.g., MBC), including platinum-resistant and/or advanced, refractory and/or recurrent forms thereof. The chemotherapeutic agent(s) can optionally be carboplatin and/or paclitaxel.

Examples of various cancer types that can be treated with a VEGF antagonist (e.g., an anti-VEGF antibody, such as bevacizumab) are listed in the definition section, above. Preferred cancer types include gynecologic cancers (e.g., ovarian, peritoneal, fallopian tube, cervical, endometrial, vaginal, and vulvar cancer). In various embodiments, the cancer that is treated is advanced, refractory, recurrent, chemotherapy-resistant, and/or platinum-resistant cancer.

Therapy with a VEGF antagonist (e.g., an anti-VEGF antibody, such as bevacizumab), optionally in combination with one or more chemotherapeutic agents (e.g., carboplatin and/or paclitaxel) preferably extends and/or improves survival, including progression free survival (PFS) and/or overall survival (OS). In one embodiment, therapy with the VEGF antagonist (e.g., an anti-VEGF antibody, such as bevacizumab) extends survival at least about 20% more than survival achieved by administering an approved anti-tumor agent, or standard of care, for the cancer being treated. In preferred embodiments, the patient has a gynecologic cancer (e.g., ovarian, peritoneal, fallopian tube, cervical, endometrial, vaginal, or vulvar cancer) or breast cancer (e.g., MBC). The patient may optionally have an advanced, refractory, recurrent, chemotherapy-resistant, and/or platinum-resistant form of the cancer.

The VEGF antagonist (e.g., an anti-VEGF antibody, such as bevacizumab), optionally in combination with one or more chemotherapeutic agents (e.g., carboplatin and/or paclitaxel), is administered to a human patient in accordance with known methods, such as intravenous administration, e.g., as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

For the prevention or treatment of cancer, the dose of VEGF antagonist (e.g., an anti-VEGF antibody, such as bevacizumab) and/or chemotherapeutic agent will depend on the type of cancer to be treated, as defined above, the severity and course of the cancer, whether the antibody is administered for preventive or therapeutic purposes, previous therapy, the patient's clinical history and response to the drug, and the discretion of the attending physician. In one embodiment, VEGF antagonist (e.g., bevacizumab) is administered at 5 mg/kg of body weight given once every 2 weeks, 10 mg/kg of body weight given once every 2 weeks , 7.5 mg/kg of body weight given once every 3 weeks, or 15 mg/kg of body weight given once every 3 weeks.

In one embodiment, a fixed dose of the VEGF antagonist is administered. The fixed dose may suitably be administered to the patient at one time or over a series of treatments. Where a fixed dose is administered, preferably it is in the range from about 20 mg to about 2000 mg of the inhibitor. For example, the fixed dose may be approximately 420 mg, approximately 525 mg, approximately 840 mg, or approximately 1050 mg of the inhibitor (e.g., an anti-VEGF antibody, such as bevacizumab). Where a series of doses are administered, these may, for example, be administered approximately every week, approximately every 2 weeks, approximately every 3 weeks, or approximately every 4 weeks, but preferably approximately every 3 weeks. The fixed doses may, for example, continue to be administered until disease progression, adverse event, or other time as determined by the physician. For example, from about two, three, or four, up to about 17 or more fixed doses may be administered.

In one embodiment, one or more loading dose(s) of the VEGF antagonist (e.g., an anti-VEGF antibody, such as bevacizumab) are administered, followed by one or more maintenance dose(s). In another embodiment, a plurality of the same dose is administered to the patient. According to one preferred embodiment of the invention, a fixed dose of a VEGF antagonist (e.g., an anti-VEGF antibody, such as bevacizumab) of approximately 840 mg (loading dose) is administered, followed by one or more doses of approximately 420 mg (maintenance dose(s)) of the antagonist. The maintenance doses are preferably administered about every 3 weeks, for a total of at least two doses, up to 17 or more doses.

According to another preferred embodiment of the invention, one or more fixed dose(s) of approximately 1050 mg of the VEGF antagonist (e.g., an anti-VEGF antibody, such as bevacizumab) are administered, for example every 3 weeks. According to this embodiment, one, two or more of the fixed doses are administered, e.g., for up to one year (17 cycles), and longer as desired.

In another embodiment, a fixed dose of approximately 1050 mg of the VEGF antagonist (e.g., an anti-VEGF antibody, such as bevacizumab) is administered as a loading dose, followed by one or more maintenance dose(s) of approximately 525 mg. About one, two, or more maintenance doses may be administered to the patient every 3 weeks according to this embodiment.

While the VEGF antagonist (e.g., an anti-VEGF antibody, such as bevacizumab) or chemotherapeutic agent may be administered as a single anti-tumor agent, the patient is optionally treated with a combination of the inhibitor (or chemotherapeutic agent), and one or more (additional) chemotherapeutic agent(s). Exemplary chemotherapeutic agents herein include: gemcitabine, carboplatin, oxaliplatin, irinotecan, fluoropyrimidine (e.g., 5-FU), paclitaxel (e.g., nab-paclitaxel), docetaxel, topotecan, capecitabine, temozolomide, interferon-alpha, and/or liposomal doxorubicin (e.g., pegylated liposomal doxorubicin). In some embodiments, at least one of the chemotherapeutic agents is carboplatin or paclitaxel. The combined administration includes co-administration or concurrent administration, using separate formulations or a single pharmaceutical formulation, and consecutive administration in either order, wherein preferably there is a time period while both (or all) active agents simultaneously exert their biological activities. Thus, the chemotherapeutic agent may be administered prior to, or following, administration of the VEGF antagonist (e.g., an anti-VEGF antibody, such as bevacizumab). In this embodiment, the timing between at least one administration of the chemotherapeutic agent and at least one administration of the VEGF antagonist (e.g., an anti-VEGF antibody, such as bevacizumab) is preferably approximately 1 month or less, and most preferably approximately 2 weeks or less. Alternatively, the chemotherapeutic agent and the inhibitor are administered concurrently to the patient, in a single formulation or separate formulations. Treatment with the combination of the chemotherapeutic agent (e.g., carboplatin and/or paclitaxel) and the VEGF antagonist (e.g., an anti-VEGF antibody, such as bevacizumab) may result in a synergistic, or greater than additive, therapeutic benefit to the patient.

Particularly desired chemotherapeutic agents for combining with the VEGF antagonist (e.g., an anti-VEGF antibody, such as bevacizumab), e.g. for therapy of ovarian cancer, include: a chemotherapeutic agent such as a platinum compound (e.g., carboplatin), a taxol such as paclitaxel or docetaxel, topotecan, or liposomal doxorubicin.

Particularly desired chemotherapeutic agents for combining with the VEGF antagonist (e.g., an anti-VEGF antibody, such as bevacizumab), e.g., for therapy of advanced stage epithelial ovarian cancer, fallopian tube cancer, or primary peritoneal cancer include: chemotherapeutic agents such as carboplatin and paclitaxel.

Particularly desired chemotherapeutic agents for combining with the VEGF antagonist (e.g., an anti-VEGF antibody such as bevacizumab), e.g., for therapy of platinum-sensitive epithelial ovarian cancer, fallopian tube cancer, or primary peritoneal cancer include: chemotherapeutic agents such as carboplatin and gemcitabine.

Particularly desired chemotherapeutic agents for combining with the VEGF antagonist (e.g., an anti-VEGF antibody such as bevacizumab), e.g., for therapy of platinum-resistant recurrent epithelial ovarian cancer, fallopian tube cancer, or primary peritoneal cancer include: a chemotherapeutic agent such as paclitaxel, topotecan, or pegylated liposomal doxorubicin.

Particularly desired chemotherapeutic agents for combining with the VEGF antagonist (e.g., an anti-VEGF antibody, such as bevacizumab), e.g., for therapy of breast cancer, include: chemotherapeutic agents such as capecitabine, and a taxol such as paclitaxel (e.g., nab-paclitaxel) or docetaxel.

Particularly desired chemotherapeutic agents for combining with the VEGF antagonist (e.g., an anti-VEGF antibody, such as bevacizumab), e.g., for therapy of glioblastoma, include: chemotherapeutic agents such as temozolomide, optionally in combination with radiotherapy.

Particularly desired chemotherapeutic agents for combining with the VEGF antagonist (e.g., an anti-VEGF antibody, such as bevacizumab), e.g., for therapy of colorectal cancer, include: chemotherapeutic agents such as a fluoropyrimidine (e.g., 5-FU), paclitaxel, cisplatin, topotecan, irinotecan, fluoropyrimidine-oxaliplatin, fluoropyrimidine-irinotecan, FOLFOX4 (5-FU, lecovorin, oxaliplatin), and IFL (ironotecan, 5-FU, leucovorin).

Particularly desired chemotherapeutic agents for combining with the VEGF antagonist (e.g., an anti-VEGF antibody, such as bevacizumab), e.g., for therapy of renal cell carcinoma, include: chemotherapeutic agents such as interferon-alpha2a.

Particularly desired chemotherapeutic agents for combining with the VEGF antagonist (e.g., an anti-VEGF antibody, such as bevacizumab), e.g., for therapy of cervical cancer, include: chemotherapeutic agents such as paclitaxel, cisplatin, topotecan, paclitaxel in combination with cisplatin, and paclitaxel in combination with topotecan.

A chemotherapeutic agent, if administered, is usually administered at dosages known therefore, or optionally lowered due to combined action of the drugs or negative side effects attributable to administration of the chemotherapeutic agent. Preparation and dosing schedules for such chemotherapeutic agents may be used according to manufacturers' instructions or as determined empirically by the skilled practitioner. Where the chemotherapeutic agent is paclitaxel, preferably, it is administered at a dose between about 130 mg/m² to 200 mg/m² (for example approximately 175 mg/m²), for instance, over 3 hours, once every 3 weeks. Where the chemotherapeutic agent is carboplatin, preferably it is administered by calculating the dose of carboplatin using the Calvert formula which is based on a patient's preexisting renal function or renal function and desired platelet nadir. Renal excretion is the major route of elimination for carboplatin. The use of this dosing formula, as compared to empirical dose calculation based on body surface area, allows compensation for patient variations in pretreatment renal function that might otherwise result in either underdosing (in patients with above average renal function) or overdosing (in patients with impaired renal function). The target AUC of 4-6 mg/mL/min using single agent carboplatin appears to provide the most appropriate dose range in previously treated patients.

Aside from the VEGF antagonist (e.g., an anti-VEGF antibody, such as bevacizumab) and chemotherapeutic agent, other therapeutic regimens may be combined therewith. For example, a second (third, fourth, etc.) chemotherapeutic agent(s) may be administered, wherein the second chemotherapeutic agent is an antimetabolite chemotherapeutic agent, or a chemotherapeutic agent that is not an antimetabolite. For example, the second chemotherapeutic agent may be a taxane (such as paclitaxel or docetaxel), capecitabine, or platinum-based chemotherapeutic agent (such as carboplatin, cisplatin, or oxaliplatin), anthracycline (such as doxorubicin, including, liposomal doxorubicin), topotecan, pemetrexed, vinca alkaloid (such as vinorelbine), and TLK 286. "Cocktails" of different chemotherapeutic agents may be administered.

Other therapeutic agents that may be combined with the VEGF antagonist (e.g., an anti-VEGF antibody, such as bevacizumab) and/or chemotherapeutic agent include any one or more of: a HER inhibitor, HER dimerization inhibitor (for example, a growth inhibitory HER2 antibody such as trastuzumab, or a HER2 antibody which induces apoptosis of a HER2-overexpressing cell, such as 7C2, 7F3 or humanized variants thereof); an antibody directed against a different tumor associated antigen, such as EGFR, HER3, HE R4; anti-hormonal compound, e.g., an anti-estrogen compound such as tamoxifen, or an aromatase inhibitor; a cardioprotectant (to prevent or reduce any myocardial dysfunction associated with the therapy); a cytokine; an EGFR-targeted drug (such as TARCEVA® IRESSA® or cetuximab); a tyrosine kinase inhibitor; a COX inhibitor (for instance a COX-1 or COX-2 inhibitor); non-steroidal anti-inflammatory drug, celecoxib (CELEBREX®); farnesyl transferase inhibitor (for example, Tipifarnib/ZARNESTRA® R115777 available from Johnson and Johnson or Lonafarnib SCH66336 available from Schering-Plough); antibody that binds oncofetal protein CA 125 such as Oregovomab (MoAb B43.13); HER2 vaccine (such as HER2AutoVac vaccine from Pharmexia, or APC8024 protein vaccine from Dendreon, or HER2 peptide vaccine from GSK/Corixa); another HER targeting therapy (e.g. trastuzumab, cetuximab, ABX-EGF, EMD7200, gefitinib, erlotinib, CP724714, CI1033, GW572016, IMC-11F8, TAK165, etc); Raf and/or ras inhibitor (see, for example, WO 2003/86467); doxorubicin HCl liposome injection (DOXIL®); topoisomerase 1 inhibitor such as topotecan; taxane; HER2 and EGFR dual tyrosine kinase inhibitor such as lapatinib/GW572016; TLK286 (TELCYTA®); EMD-7200; a medicament that treats nausea such as a serotonin antagonist, steroid, or benzodiazepine; a medicament that prevents or treats skin rash or standard acne therapies, including topical or oral antibiotic; a medicament that treats or prevents diarrhea; a body temperature-reducing medicament such as acetaminophen, diphenhydramine, or meperidine; hematopoietic growth factor, etc.

Suitable dosages for any of the above-noted co-administered agents are those presently used and may be lowered due to the combined action (synergy) of the agent and inhibitor. In addition to the above therapeutic regimes, the patient may be subjected to surgical removal of tumors and/or cancer cells, and/or radiation therapy.

Where the VEGF antagonist is an antibody (e.g., bevacizumab), preferably the administered antibody is a naked antibody. The VEGF antagonist (e.g., an anti-VEGF antibody, such as bevacizumab) administered may be conjugated with a cytotoxic agent. Preferably, the conjugated and/or antigen to which it is bound is/are internalized by the cell, resulting in increased therapeutic efficacy of the conjugate in killing the cancer cell to which it binds. In a preferred embodiment, the cytotoxic agent targets or interferes with nucleic acid in the cancer cell. Examples of such cytotoxic agents include maytansinoids, calicheamicins, ribonucleases, and DNA endonucleases.

The VEGF antagonist (e.g., an anti-VEGF antibody, such as bevacizumab) can be administered by gene therapy. See, for example, WO 96/07321 published Mar. 14, 1996 concerning the use of gene therapy to generate intracellular antibodies. There are two major approaches to getting the nucleic acid (optionally contained in a vector) into the patient's cells; in vivo and ex vivo. For in vivo delivery the nucleic acid is injected directly into the patient, usually at the site where the antibody is required. For ex vivo treatment, the patient's cells are removed, the nucleic acid is introduced into these isolated cells and the modified cells are administered to the patient either directly or, for example, encapsulated within porous membranes which are implanted into the patient (see, e.g. U.S. Pat. Nos. 4,892,538 and 5,283,187). There are a variety of techniques available for introducing nucleic acids into viable cells. The techniques vary depending upon whether the nucleic acid is transferred into cultured cells in vitro, or in vivo in the cells of the intended host. Techniques suitable for the transfer of nucleic acid into mammalian cells in vitro include the use of liposomes, electroporation, microinjection, cell fusion, DEAE-dextran, the calcium phosphate precipitation method, etc. A commonly used vector for ex vivo delivery of the gene is a retrovirus. The currently preferred in vivo nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu et al., J. Biol. Chem. 262:44294432 (1987); and Wagner et al., Proc. Natl. Acad. Sci. USA 87:3410-3414 (1990). For review of the currently known gene marking and gene therapy protocols see Anderson et al., Science 256:808-813 (1992). See also WO 93/25673 and the references cited therein.

### B. Methods of Prognosis, Diagnosis and Detection

The present invention provides methods for improving the progression-free survival (PFS) and overall survival (OS) of patients suffering from cancer (e.g., a gynecologic cancer (e.g., ovarian, peritoneal, fallopian tube, cervical, endometrial, vaginal, or vulvar cancer), or breast cancer (e.g., MBC; also see below)), by treatment with a VEGF antagonist (e.g., an anti-VEGF antibody, such as bevacizumab), optionally in combination with a chemotherapy regimen. The invention also provides methods of identifying a patient suffering from a cancer (e.g., a gynecologic cancer (e.g., ovarian, peritoneal, fallopian tube, cervical, endometrial, vaginal, or vulvar cancer) or breast cancer (e.g., metastatic breast cancer (MBC); also see below)) who may benefit from administration of a VEGF antagonist (e.g., an anti-VEGF antibody, such as bevacizumab), optionally in combination with a chemotherapy regiment. These methods involve determining the expression level of CD31 and/or tumor VEGFA and comparing them to median levels in the cancer type, where expression of CD31 and/or tumor VEGFA at a level more than the median level for expression in the cancer type indicates that the patient may benefit from administration of a VEGF antagonist (e.g., an anti-VEGF antibody, such as bevacizumab), optionally in addition to another anti-cancer therapy (e.g., a chemotherapy regimen (e.g., carboplatin and/or paclitaxel)).

The present invention further provides for methods for assessing the sensitivity or responsiveness of a patient to a VEGF antagonist (e.g., an anti-VEGF antibody, such as bevacizumab), optionally in combination with a chemotherapy regimen, by determining the expression level of one or more of CD31 and/or tumor VEGFA relative to control levels in patients diagnosed with cancer (e.g., a gynecologic cancer (e.g., ovarian, peritoneal, fallopian tube, cervical, endometrial, vaginal, or vulvar cancer), or breast cancer (e.g., metastatic breast cancer (MBC); also see below)).

The present invention further provides methods of prognosis of a patient suffering from cancer (e.g., a gynecologic caner (e.g., ovarian, peritoneal, fallopian tube, cervical, endometrial, vaginal, or vulvar cancer) or breast cancer (e.g., metastatic breast cancer (MBC); also see below)) by determining the expression level of CD31 and/or tumor VEGFA in a sample obtained from the patient, comparing the level of CD31 MVD and/or tumor expression VEGFA to the median level for CD31 MVD and/or tumor VEGFA expression, respectively, in the cancer type, and determining a prognosis for the patient. A poor prognosis is when expression of CD31 MVD and/or tumor VEGFA is at a level more than the median level for CD31 and/or tumor VEGFA expression. The methods optionally include the step of identifying the patient as likely to benefit from administration of a VEGF antagonist (e.g., an anti-VEGF antibody, such as bevacizumab) when the patient is determined to have a poor prognosis of survival, and further optionally include the step of administering a VEGF antagonist (e.g., an anti-VEGF antibody, such as bevacizumab) in a therapeutically effective amount to the patient, if the patient is determined to have a poor prognosis.

Accordingly, the present invention relates to the identification, selection, and use of biomarkers of cancer (e.g., a gynecologic cancer (e.g., ovarian, peritoneal, fallopian tube, cervical, endometrial, vaginal, or vulvar cancer) or breast cancer (e.g., MBC; also see above)) that associate with sensitivity or responsiveness to angiogenesis inhibitors, e.g., VEGF antagonists (e.g., anti-VEGF antibodies, such as bevacizumab), optionally in combination with chemotherapeutic regimens, such as carboplatin-based chemotherapies. In this respect, the invention relates to the use of (a) tumor specific expression profile(s) of one or more of CD31 and/or tumor VEGFA relative to controls (e.g., the median) established in patients diagnosed with cancer (e.g., a gynecologic cancer (e.g., ovarian, peritoneal, fallopian tube, cervical, endometrial, vaginal, or vulvar cancer) or breast cancer (e.g., MBC; also see above)), to identify patients sensitive or responsive to the addition of angiogenesis inhibitors, for example, VEGF antagonists (e.g., anti-VEGF antibodies, such as bevacizumab), to standard chemotherapies. The invention further relates to methods for improving PFS and/or OS of a patient suffering from cancer (e.g., a gynecologic cancer (e.g., ovarian, peritoneal, fallopian tube, cervical, endometrial, vaginal, or vulvar cancer) or breast cancer (e.g., MBC; also see above)), by the addition of angiogenesis inhibitors, for example, VEGF antagonists (e.g., anti-VEGF antibodies, such as bevacizumab), to standard chemotherapies, e.g., carboplatin- and/or paclitaxel-based chemotherapies, by determining (a) tumor specific expression level(s) of one or more of CD31 and/or tumor VEGFA relative to control(s) (e.g., the median) in patients diagnosed with cancer (e.g., a gynecologic cancer (e.g., ovarian, peritoneal, fallopian tube, cervical, endometrial, vaginal, or vulvar cancer) or breast cancer (e.g., MBC; also see above)).

The expression level of CD31 and/or tumor VEGFA may be assessed by any method known in the art suitable for determination of specific protein levels in a patient sample, and is preferably determined by an immunohistochemical ("IHC") method employing antibodies specific for CD31 and/or tumor VEGFA. Such methods are well known and routinely implemented in the art, and corresponding commercial antibodies and/or kits are readily available. For example, commercially available antibodies/test kits for VEGFA and CD31 can be obtained from Abcam, Inc. (Cambridge, Mass., U.S.A.) as clone SP28, and from Dako A/S (Glostrup, Denmark), as clone JC70A, respectively. Preferably, the expression levels of the marker/indicator proteins of the invention are assessed using the reagents and/or protocol recommendations of the antibody or kit manufacturer. The skilled person will also be aware of further means for determining the expression level of CD31 and/or tumor VEGFA by IHC methods. Therefore, the expression level of one or more of the markers/indicators of the invention can be routinely and reproducibly determined by a person skilled in the art without undue burden. However, to ensure accurate and reproducible results, the invention also encompasses the testing of patient samples in a specialized laboratory that can ensure the validation of testing procedures.

Preferably, the expression level of CD31 and/or tumor VEGFA is assessed in a biological sample that contains or is suspected to contain cancer cells. The sample may be, for example, an ovarian tissue resection, an ovarian tissue biopsy, or a metastatic lesion obtained from a patient suffering from, suspected to suffer from, or diagnosed with cancer (e.g., a gynecologic cancer, in particular ovarian cancer). Preferably, the sample is a sample of ovarian tissue, a resection or biopsy of an ovarian tumor, a known or suspected metastatic ovarian cancer lesion or section, or a blood sample, e.g., a peripheral blood sample, known or suspected to comprise circulating cancer cells, e.g., ovarian cancer cells. The sample may comprise both cancer cells, i.e., tumor cells, and non-cancerous cells, and, in certain embodiments, comprises both cancerous and non-cancerous cells. In aspects of the invention comprising the determination of vessel number in a sample (see, e.g., examples below), the sample comprises both cancer/tumor cells and non-cancerous cells that are endothelial cells. The skilled artisan, e.g., a pathologist, can readily discern cancer cells from non-cancerous, e.g., endothelial cells, as well as determine vessel number within a sample, e.g., by staining the sample for detection of an endothelial cell marker, e.g., CD31. As an alternative or additional to direct determination of vessel number, the expression level of one or more endothelial cell markers, e.g., CD31, may also be determined, which level correlates with vessel number. Methods of obtaining biological samples including tissue resections, biopsies, and body fluids, e.g., blood samples comprising cancer/tumor cells, are well known in the art. In someembodiments, the sample obtained from the patient is collected prior to beginning any chemotherapeutic regimen or therapy, e.g., therapy for the treatment of cancer or the management or amelioration of a symptom thereof. Therefore, in some embodiments, the sample is collected before the administration of chemotherapeutics or the start of a chemotherapy regimen.

In addition to the methods described above, the invention also encompasses further immunohistochemical methods for assessing the expression level of one or more of tumor CD31 and/or tumor VEGFA, such as by Western blotting and ELISA-based detection. Similar methods may be employed in alternative or additional methods for the determination of vessel number, including the determination of tumor specific expression level of one or more endothelial cell markers, e.g., CD31. As is understood in the art, the expression level of the marker/indicator proteins of the invention may also be assessed at the mRNA level by any suitable method known in the art, such as Northern blotting, real time PCR, and RT PCR. Immunohistochemical- and mRNA-based detection methods and systems are well known in the art and can be deduced from standard textbooks, such as Lottspeich (Bioanalytik, Spektrum Akademisher Verlag, 1998) or Sambrook and Russell (Molecular Cloning: A Laboratory Manual, CSH Press, Cold Spring Harbor, N.Y., U.S.A., 2001). The described methods are of particular use for determining the expression levels of CD31 and/or tumor VEGFAin a patient or group of patients relative to control levels established in a population diagnosed with advanced stages of a cancer (e.g., a gynecologic cancer, such as ovarian cancer).

The expression level of one or more of VEGFA and/or one or more endothelial cell markers, e.g., CD31, can also be determined on the protein level by taking advantage of immunoagglutination, immunoprecipitation (e.g., immunodiffusion, immunelectrophoresis, immune fixation), western blotting techniques (e.g., (in situ) immuno histochemistry, (in situ) immuno cytochemistry, affinitychromatography, enzyme immunoassays), and the like. Amounts of purified polypeptide in solution may also be determined by physical methods, e.g. photometry. Methods of quantifying a particular polypeptide in a mixture usually rely on specific binding, e.g., of antibodies.

As mentioned above, the expression level of the marker/indicator proteins according to the present invention may also be reflected in a decreased expression of the corresponding gene(s) encoding the VEGFA and/or one or more endothelial cell markers, e.g., CD31, for determination of vessel number as described herein. Therefore, a quantitative assessment of the gene product prior to translation (e.g. spliced, unspliced or partially spliced mRNA) can be performed in order to evaluate the expression of the corresponding gene(s). The person skilled in the art is aware of standard methods to be used in this context or may deduce these methods from standard textbooks (e.g. Sambrook, 2001, loc. cit.). For example, quantitative data on the respective concentration/amounts of mRNA encoding one or more of VEGFA and/or one or more endothelial cell markers, e.g., CD31, for determination of vessel number as described herein can be obtained by Northern Blot, Real Time PCR, and the like.

For use in the detection methods described herein, the skilled person has the ability to label the polypeptides or oligonucleotides encompassed by the present invention. As routinely practiced in the art, hybridization probes for use in detecting mRNA levels and/or antibodies or antibody fragments for use in IHC methods can be labelled and visualized according to standard methods known in the art. Non-limiting examples of commonly used systems include the use of radiolabels, enzyme labels, fluorescent tags, biotin-avidin complexes, chemiluminescence, and the like.

As an alternative or in addition to the determination of the expression level of one or more of CD31 and tumor VEGFA according to the methods described herein, the vessel number in a tumor sample, relative to (a) control level(s) established in patients diagnosed with cancer (e.g., a gynecologic cancer (e.g., ovarian, peritoneal, fallopian tube, cervical, endometrial, vaginal, or vulvar cancer), or breast cancer (e.g., MBC; also see above)), can be determined as a biomarker and an indicator of a patient sensitive or responsive to angiogenesis inhibitors, for example, VEGF antagonists (e.g., anti-VEGF antibodies, such as bevacizumab), optionally in addition to standard chemotherapies.

### EXAMPLES

### CD31 and tumor VEGFA as Predictive Biomarkers for Improved Bevacizumab Efficacy in First Line Ovarian Cancer: A Tumor Tissue Retrospective Analysis

### A. Samples and Subjects

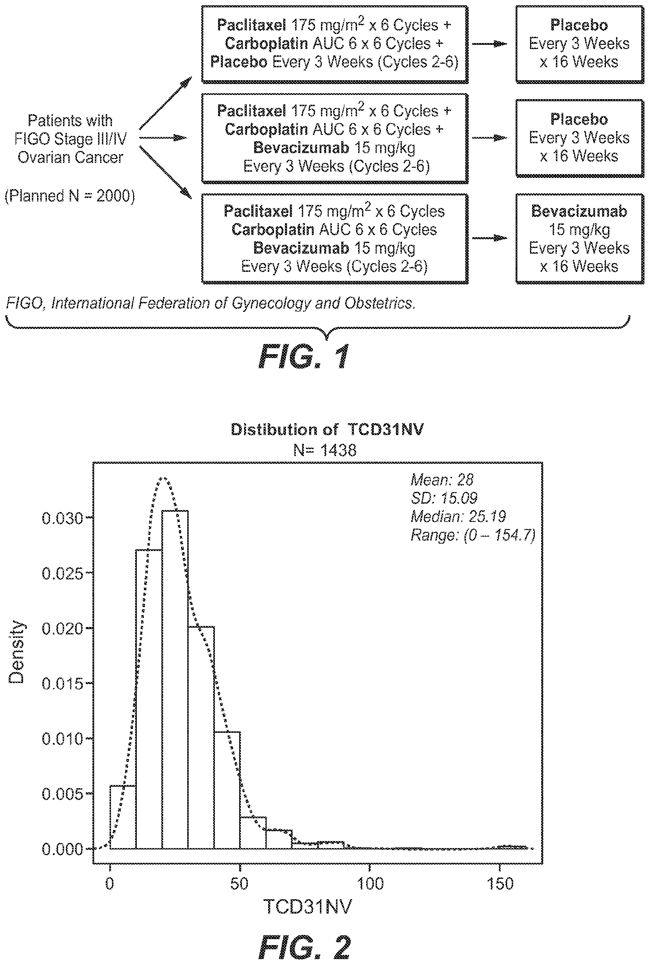

Tumor tissue samples were collected from patients with newly diagnosed, previously untreated, suboptimal advanced stage epithelial ovarian and primary peritoneal cancer, and participating in a phase III trial of carboplatin and paclitaxel plus placebo (CPP), versus carboplatin and paclitaxel plus concurrent bevacizumab followed by placebo (CPB15), versus carboplatin and paclitaxel plus concurrent and extended bevacizumab (CPT15+) (Fig. 1). The intended to treat (ITT) population analysis included 1,852 patients and the biomarker evaluable population (BEP) population analysis included 1,438 patients.

### B. Analytical Methods

Analytical results from this study were generated using standardized statistical tools to address the following questions:
1.) Representativeness of the Biomarker Population: Key baseline demographics and prognostic characteristics (including stratification variables and any variables with known prognostic significance) and efficacy outcomes were summarized by treatment groups and compared between biomarker and ITT populations to investigate any potential selection bias associated with the availability of the biomarker.
2.) Biomarker Properties: Overall distribution of the biomarker at baseline was plotted and descriptive statistics (mean, standard deviation, and range) were used to summarize baseline biomarker values for the patient population. The relationships between the biomarker and key demographic prognostic variables were investigated using bivariate plots. Prognostic properties of the biomarkers were assessed by estimates of the clinical efficacy in the control arm and corresponding 95% confidence intervals were tabulated.
3.) Predictive Properties of the Biomarker: The predictive effects of the biomarker measured at baseline were evaluated using exploratory graphics. STEPP (subgroup treatment effect pattern plot) for continuous biomarkers and Forest plot at pre-specified cutoffs (quartiles) were the primary outputs to explore cutoff selection.
4.) Subgroup Analysis: Once the cutoff was determined, the predictive effects of the biomarkers measured at baseline were evaluated using a statistical model with an interaction term and by comparing the treatment effect in the two groups of patients defined by the pre-specified value of the biomarker. Standardized outputs for Kaplan-Meier curves and Forest plots were provided. In addition, selection bias between the arms within biomarker high or low groups were assessed.

### C. Results

### Patient Demographics

For a given biomarker, the BEP population includes all subjects who were randomized and having non-missing biomarker data at baseline. The non-biomarker population is defined as a complement to the biomarker evaluable population. For this study, the BEP includes patients with non-missing CD31 RNA data. Key demographics and baseline characteristics (including stratification variables and any variables with known prognostic significance) and efficacy outcomes were summarized by treatment groups and compared between biomarker and intended to treat (ITT) populations to investigate any potential selection biases associated with the missing status of the biomarker. In the present study, the progression free survival (PFS) and overall survival (OS) between the ITT (N=1852) and BEP (N=1438) populations were comparable. The details of the patient demographics in the ITT and BEP populations are shown in Table 1.

**Table 1. ITT vs. BEP Patient Demographics**

| | CPP | | CPB15 | | CPB15+ | |
|---|---|---|---|---|---|---|
| | ITT | TCD31NV | ITT | TCD31NV | ITT | TCD31NV |
| **GOG Performance Status** | | | | | | |
| Total | 625 | 483 | 625 | 463 | 623 | 492 |
| 0 | 311 (49.76%) | 234 (48.45%) | 314 (50.24%) | 242 (52.27%) | 307 (49.28%) | 246(50%) |
| 1 or 2 | 314 (50.24%) | 249 (51.55%) | 311 (49.76%) | 221 (47.73%) | 316 (50.72%) | 246(50%) |

| **Stage** | | | | | | |
|---|---|---|---|---|---|---|
| Total | 625 | 483 | 625 | 463 | 623 | 492 |
| Stage III Optimally Debulked | 219 (35.04%) | 163 (33.75%) | 204 (32.64%) | 152 (32.83%) | 216 (34.67%) | 165 (33.54%) |
| Stage III Sub-optimally Debulked | 253 (40.48%) | 200 (41.41%) | 256 (40.96%) | 183 (39.52%) | 242 (38.84%) | 200 (40.65%) |
| Stage IV | 153 (24.48%) | 120 (24.84%) | 165 (26.4%) | 128 (27.65%) | 265 (26.48%) | 127 (25.81%) |

| **Residual Disease Size** | | | | | | |
|---|---|---|---|---|---|---|
| N | 625 | 483 | 625 | 463 | 623 | 492 |
| Mean | 2.57 | 2.69 | 2.5 | 2.59 | 2.69 | 2.67 |
| SD | 3.49 | 3.66 | 3.5 | 3.78 | 3.77 | 3.68 |
| Median | 1.5 | 1.5 | 1.5 | 1.4 | 1.5 | 1.5 |
| Min-Max | 0...30 | 0...30 | 0...30 | 0...30 | 0...30 | 0...30 |

| **Ascites** | | | | | | |
|---|---|---|---|---|---|---|
| Total | 625 | 483 | 625 | 463 | 623 | 492 |
| UNKNOWN | 17 (2.72%) | 13 (2.69%) | 24 (3.84%) | 17 (3.67%) | 13 (2.09%) | 11 (2.24%) |
| NO | 154 (24.64%) | 121 (25.05%) | 141 (22.56%) | 101 (21.81%) | 165 (26.48%) | 133 (27.03%) |
| YES | 454 (72.64%) | 349 (72.26%) | 460 (73.6%) | 345 (74.51%) | 445 (71.43%) | 348 (70.73%) |

| **Measureable Disease** | | | | | | |
|---|---|---|---|---|---|---|
| Total | 625 | 483 | 625 | 463 | 623 | 492 |
| NO | 229 (36.64%) | 162 (33.54%) | 232 (37.12%) | 173 (37.37%) | 220 (35.31%) | 176 (35.77%) |
| YES | 396 (63.36%) | 321 (66.46%) | 393 (62.88%) | 290 (62.63%) | 403 (64.69%) | 316 (64.23%) |

| **Baseline CA-125** | | | | | | |
|---|---|---|---|---|---|---|
| Total | 625 | 483 | 625 | 463 | 623 | 492 |
| Abnormal | 590 (94.4%) | 457 (94.62%) | 586 (93.76%) | 439 (94.82%) | 592 (95.02%) | 467 (94.92%) |
| Normal | 35 (5.6%) | 26 (5.38%) | 39 (6.24%) | 24 (5.18%) | 31 (4.98%) | 25 (5.08%) |

### CD31 Immunohistochemical Methods

Immunohistochemical staining of CD31 was used for detecting blood vessels. CD31 stains endothelium from different types of vessels, including lymphatic vessels and hepatic sinusoidal endothelial cells. The shape of the CD31-stained structures ranges from thread-like (longitudinal sectioned capillaries) to single cell-like (cross-sectioned capillaries). The immunohistochemical detection of CD31 (PECAM-1) was carried out using the Ventana Benchmark@ XT platform (Ventana, Tucson, Ariz. USA). Detection of CD31 was developed by using a mouse monoclonal antibody (Clone 1A10) from Ventana. Immunohistochemistry is a semi-quantitative method used to detect presence or absence of a target antigen (e.g., CD31) in tissue, in this case formalin-fixed, paraffin-embedded tissue. In brief, protocol number 91 using the ultra view ™ Universal DAB detection kit was used. After deparaffinization and rehydration, antigen retrieval was performed by 32 minutes of anti-CD31 antibody incubation at 37°C. Validation reports showing accuracy, specificity, linearity, and precision (reproducibility and repeatability) were prepared for each IHC assay. Staining of external control slides and intrinsic control elements was documented. These methods are described in more detail, as follows.

CD31 vascular staining was evaluated by determination of the number of vessels (# per mm²) and the volume fraction (as %) of the vessels in fixed tissue samples. Generally, a stereology-based method was used for systematically uniform random sampling of fields to score for the CD31 stainings. A minimum of three regions of interest (ROIs) were selected with a maximum of 15 ROIs at 20x magnification or at 40x magnification.

Grids were placed on top of the images to allow evaluation. The selected ROI images were then combined with the predefined grids. The volume fraction was estimated by counting the grid points, formed by the crossing lines of the counting grid that fall on top of the tissue, cells, or structures of interest. This fraction was representative for the volume density of the tissue, cells, or structures of interest. Typically, only the top right corner of a cross (or grid point) was considered. Grid points that lie within obvious tissue tears or outside the tumor tissue were not counted. Small necrotic zones within tumor structures were counted because they were considered a part of the tumor. Grid points that lie within the vascular lumen were also counted. Only grids in which >75% of the area consists of tissue (e.g., ≥ 19 out of 25 grid points or ≥61 out of 81 grid points), were analyzed. If this was not the case, the region was omitted from the analysis.

A grid point was counted for Vvessel (the amount of grid points that represent vessels) when a stained endothelial cell or the vessel lumen was found to lie within the top right grid point corner. When only a single endothelial cell of a large vessel was stained, all other endothelial cells that made up this vessel were counted for Vvessel when they filled the top right corner of a grid point. A grid point was counted for V (the amount of grid points that represent other cell populations or structures) when a stained cell or structure of interest filled up the top right grid point corner. Since other cell populations or structures of interest can vary depending on the staining, the other cells or structures counted for V were defined in the scoring form.

For estimation of N (number of vessels), the same grid was used as for the determination of volume fractions. The outer borders of the raster grid delineate the counting chamber that has a given area. Vascular structures that cross the left or bottom line of the grid were not counted. If a vascular structure that was only stained within the grid but clearly the vessels crossed the border, it was not counted. In tissue sections, vessels with a clear lumen were counted. Stained structures without lumina that have a vascular appearance were also counted. For some staining assays, a further distinction between weak and strong staining intensity was required.

### Tumor VEGFA Immunohistochemical Methods

The immunohistochemical detection of tumor VEGFA was carried out using the Autostainer and PT-module instruments. The primary antibody used to detect tumor VEGFA was R547 (abcam-ab27620 (HGX-R547). The antibody is a prediluted rabbit monoclonal antibody (SP28) raised against the N-terminal part of human VEGFA. The immunogen sequence used shares about 30-40% identity to other VEGF isoforms including VEGF-B, VEGF-C, and VEGF-D. Paraffin embedded tissues were stained for tumor VEGFA by following the HistoGeneX staining protocol. The lowest positivity that is dectected/scored was a pre-set detection limit which was determined by a specific scoring system and was set as 1% of cells that stain with the weakest staining intensity 1+, further described below. To ensure that the staining was performed in a linear area, experiments were not performed with different concentrations of the primary antibody. The N-universal negative control rabbit IgG from Dako (N1699) was used as a negative control. The IgG control was checked for VEGFA immunoreactivity and was VEGFA negative, whereas the positive samples showed clear and intense cytoplasmic VEGFA positivity in the tumor cells and in syncitiotrophoblast in human placenta. Only one negative external control per staining run was performed.

The tumor VEGFA stainings were evaluated using a system in which the percentage of cells showing membrane, cytoplasmic, or nuclear staining were scored in combination with their staining intensity. Final results were extracted from the scoring matrix and reported. Depending on the protocol, the part of the tissue needed to be evaluated was determined, for example, invasive part of the tumor, entire tumor, carcinoma in situ, etc. In addition, depending on the protocol, the cell compartment needed to be scored was determined (e.g., membrane, cytoplasmic, or nuclear). In general, the cell compartments were scored separately.

The scoring included the evaluation of the intensity of the staining and the proportion of the cells staining positively at any intensity. For the membrane staining, the pattern of full basolateral or complete staining was also reported. The staining signal was divided into four different intensity categories: 0= no staining; 1+=weak staining (visible at high power magnification); 2+=intermediate (or moderate) staining (visible at low power magnification); 3+=strong staining (striking even at low power magnification).

For each intensity, the proportion (percentage) of staining was scored. The maximum percentage of all intensities together (i.e., the sum of all percentages at all intensities) must be 100%. In general, the rule of thumb to assign intensity and percentages of the staining is: a) identify the most dominant staining intensity and assign a certain % to it; b) define the % of the lowest staining; c) divide the rest of the % between the lowest and the highest category; d) when staining positivity is between 0 and 10% accuracy of 1% is recommended; e) when staining positivity is more than 10%, accuracy of 5% is recommended. The number of fields scored (ROI) was selected to be optimal (for large tissue samples a minimum of 10 ROI's is recommended) and selected systematically uniform random according to the stereology-based random sample method. The percentage of tumor cells showing each of membrane staining, cytoplasmic staining, and nuclear staining were scored using the 0, 1+, 2+, 3+ scale, and with the total being 100%. Depending on the specific protocol, additional scores for the characterization of the staining pattern were also used, for example, the completeness of membrane staining. When membrane percentage was scored, the percentage of full basolateral or complete membrane staining was estimated for each intensity. Full basolateral staining is only seen in well-differentiated adenocarcinoma. Full basolateral staining refers to the staining of the base and two sides of the cells.

The final data set was based on the observed results as outlined above. For other purposes, recalculations from the final data can be made to generate an H-score, IRS-score or Allred score. These scoring systems combine both proportion and intensity scoring to produce categories for interpreting the effectiveness of a therapy. The recalculations of these different scoring methods are as follows and can be included in the database:
H-score: for the H-score, the percentage is multiplied by the staining intensity. The highest H-score produced is 300. The positivity is then categorized in four classes, interpreted as follows: 0-50=negative; 51-100=weak positive; 101-200=moderate positive; 201-300=strong positive.

IRS-score: the IRS-score is the sum of the highest percentage of positivity observed and multiplied with the score of its staining intensity. The highest IRS-score produced is 12. The positivity is then categorized in five classes, interpreted as follows: 0-1=negative; 2-3=weak positive; 4-8=moderate positive; 9-12=strong positive.

Allred score/Quick-score: the Allred-score or Quick-score is the sum of the score with the highest positive percentage with the score of its staining intensity. The highest Allred-score or Quick-score produced is 8. The interpretation is then categorized into two classes for the Allred-score: 0-2=negative; 3-8=positive and four classes for the Quick-score: 0-1=negative; 2-3=weak positive; 4-6=moderate positive; 7-8=strong positive.

### Properties of the Biomarker

An investigation of tumor tissue immunohistochemical (IHC) biomarkers related to angiogenesis and tumorigenesis is outlined in Table 2. The IHC markers evaluated revealed that increased expression levels of two particular biomarkers, CD31 and tumor VEGFA, correlated with improved results in subjects receiving carboplatin and paclitaxel plus concurrent and extended bevacizumab.

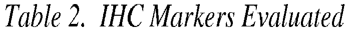

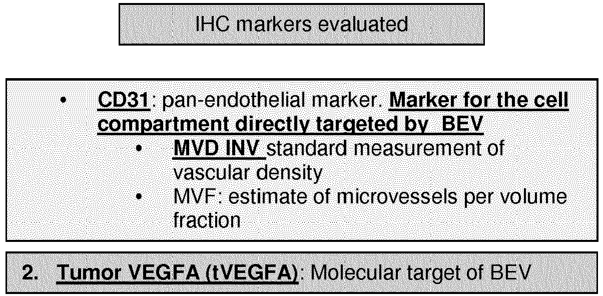

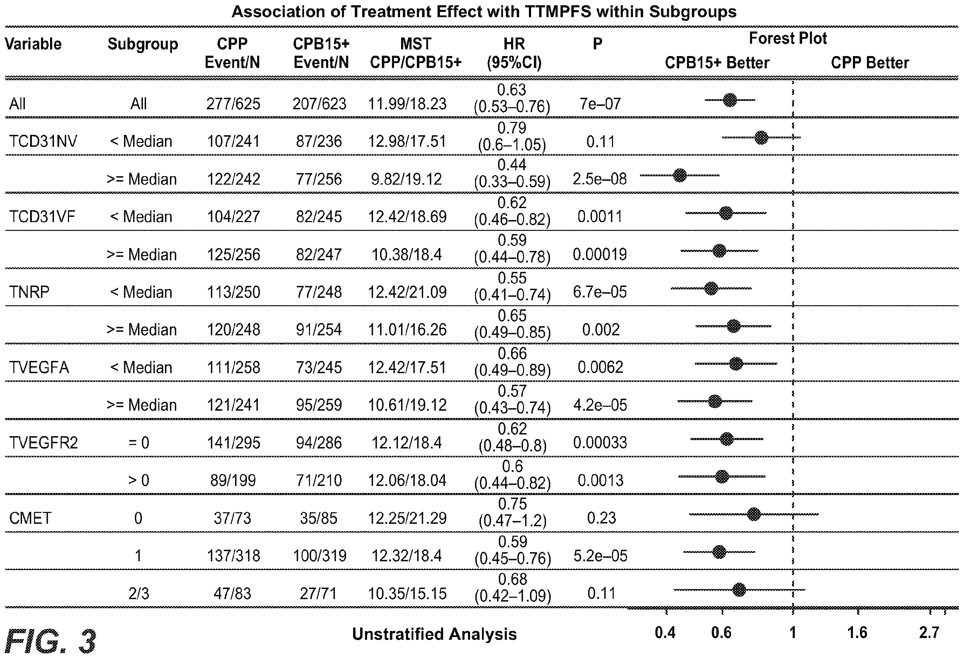

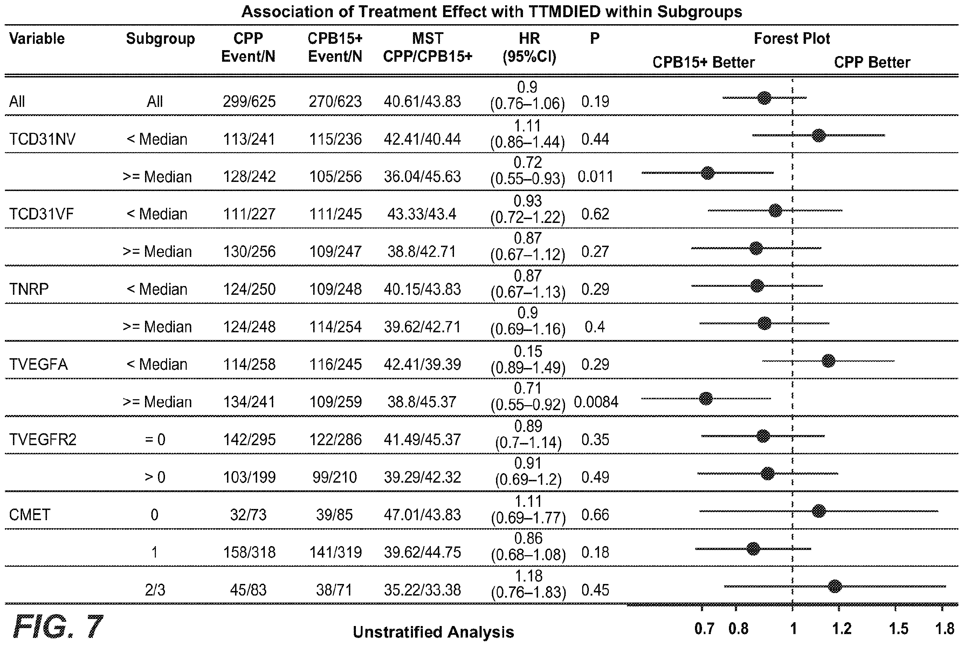

Overall distribution of CD31 at baseline was plotted and descriptive statistics (e.g., mean, standard deviation, median, and range) were used to summarize baseline biomarker values for the patient population (Fig. 2). Statistical analysis and Forest plots of PFS in subgroups of patients according to tumor cell biomarker expression of CD31 and other biomarkers at specified cutoffs are shown in Fig. 3. Statistical analysis and Forest plots of OS in subgroups of patients according to tumor cell biomarker expression of CD31 and other biomarkers at specified cutoff are shown in Fig. 7. With respect to the association of treatment effect on PFS within biomarker subgroups, the hazard ratios indicate that all patient subgroups gained benefit from bevacizumab treatment (Fig. 3). With respect to the association of treatment effect on OS within the CD31 and tumor VEGFA biomarker subgroups, the hazard ratios indicate that patient subgroups having high levels of CD31 and/or tumor VEGFA (i.e., above median or above a 50% cutoff) gained benefit from bevacizumab treatment, whereas patient subgroups having low levels of CD31 and/or tumor VEGFA (i.e., below median or below a 50% cutoff) did not benefit with the addition of bevacizumab (Fig. 7).

### Treatment and CD31 Biomarker Effect

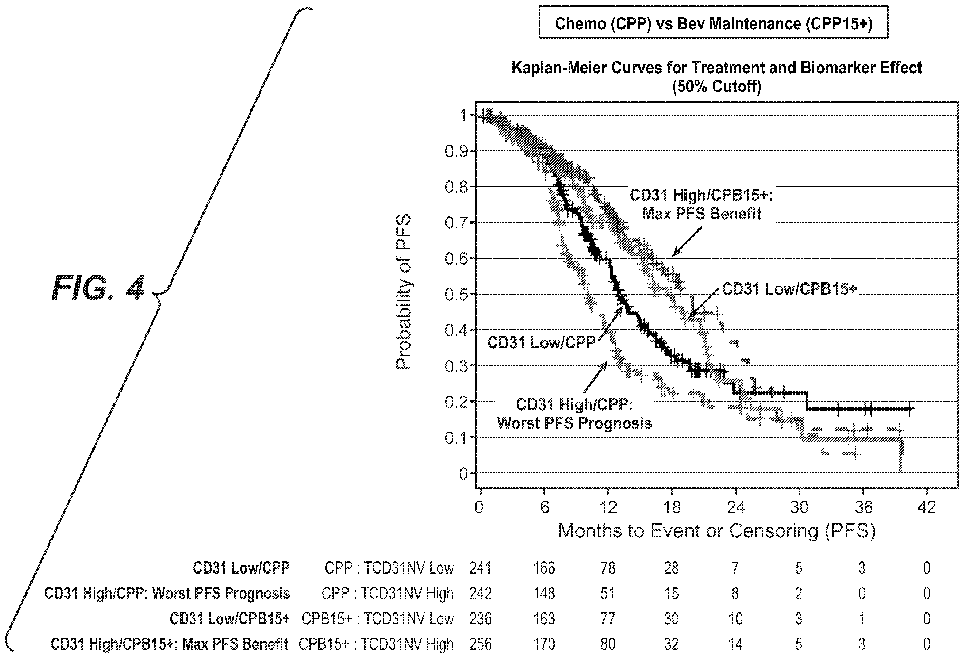

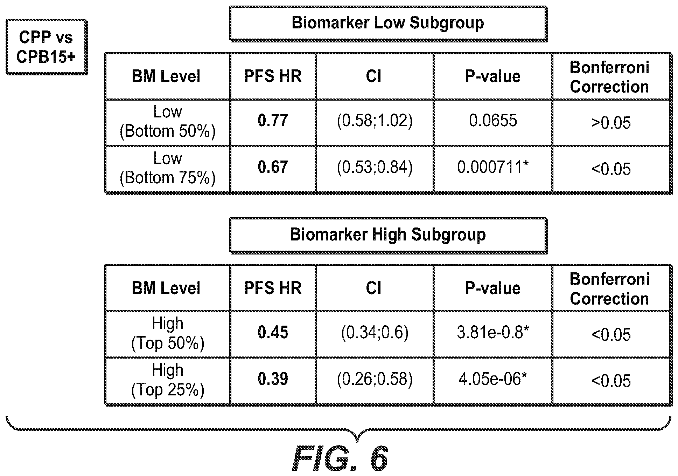

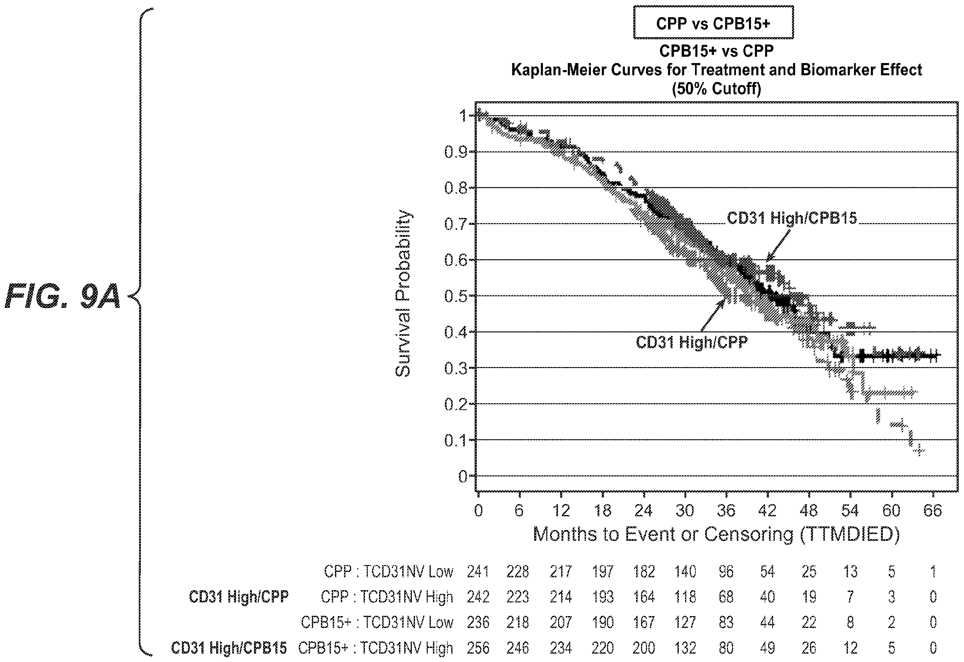

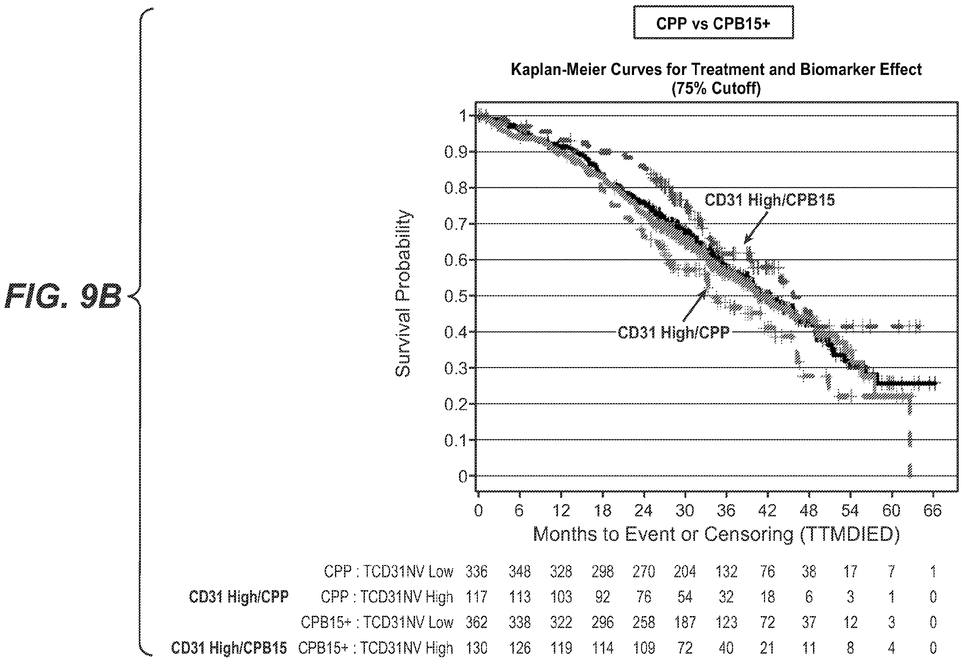

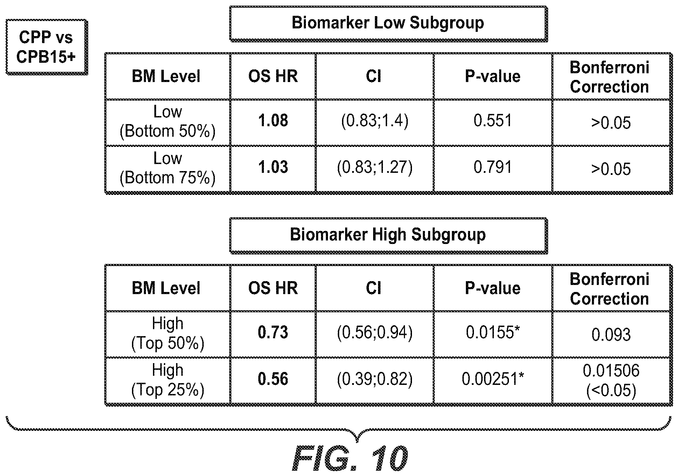

The expression of CD31 within the BEP population was further analyzed and is shown in Figs. 4-6 and 8-11. Consistent with the results shown in Fig. 3 of PFS effects in the CD31 biomarker subgroup, quartile analysis of patient subgroups based on % cutoff confirmed that as the level of CD31 expression increased, PFS also improved with bevacizumab treatment (e.g., as indicated by the hazard ratio) (Figs. 5 and 6). Kaplan-Meier curves for probability of PFS for the CD31 subgroups at 50% cutoff are shown in Fig. 4. Patients expressing high CD31 levels exhibited the maximum PFS benefit from treatment with bevacizumab, whereas the same patient population if treated with a carboplatin-paclitaxel chemotherapy regimen alone was shown to have the worst PFS prognosis. Patients expressing low CD31 levels also derived some PFS benefit with bevacizumab as compared to patients expressing low CD31 levels treated with carboplatin-paclitaxel alone. Similarly, consistent with the results shown in Fig. 7 of OS effects in the CD31 biomarker subgroup, quartile analysis of patient subgroups based on % cutoff confirmed that as the level of CD31 expression increases, a marked improvement in OS is seen with bevacizumab treatment (Figs. 8 and 10) with the biggest effect seen with the 75% cutoff. Kaplan-Meier curves for survival probability for the CD31 subgroups defined by 50% (Fig. 9A) and 75% (Fig. 9B) cutoffs confirms the enhanced bevacizumab treatment effect seen in patients expressing high CD31 at the 75% cutoff (Fig. 9B).

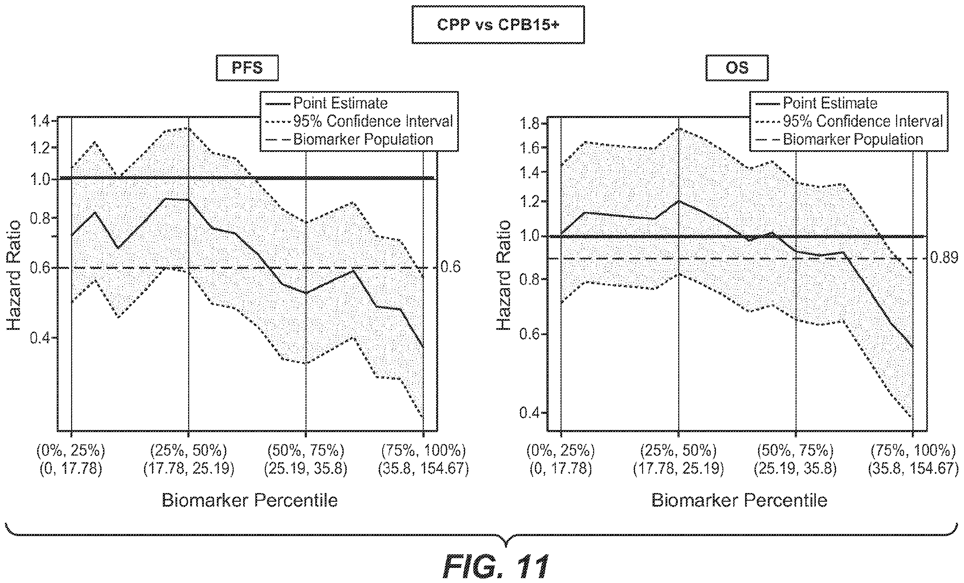

The STEPP analysis shown in Fig. 11 uses a slide window approach to demonstrate the association of CD31 expression level with hazard ratios for PFS and OS. For PFS, the hazard ratios for patients included in each subgroup (represented by middle solid line) were below 1.0, indicating that all patients derive some benefit from bevacizumab treatment. However, patients expressing high levels of CD31 (the top 50% of the CD31 expression) derived greater benefit from bevacizumab treatment. With respect to OS, the hazard ratio for patients above -75% of the CD31 expression range (represented by middle solid line) were below 1.0, indicating that the greater benefit is seen only in patients above -75% of the CD31 expression range. Together, the results suggest that higher CD31 levels (i.e., above median or above 50% of the CD31 expression range) are associated with both longer PFS and OS in bevacizumab treated patients. The data thus support CD31 as a predictive biomarker for improved PFS and OS and increased bevacizumab efficacy in ovarian cancer.

### Treatment and tumor VEGFA Biomarker Effect

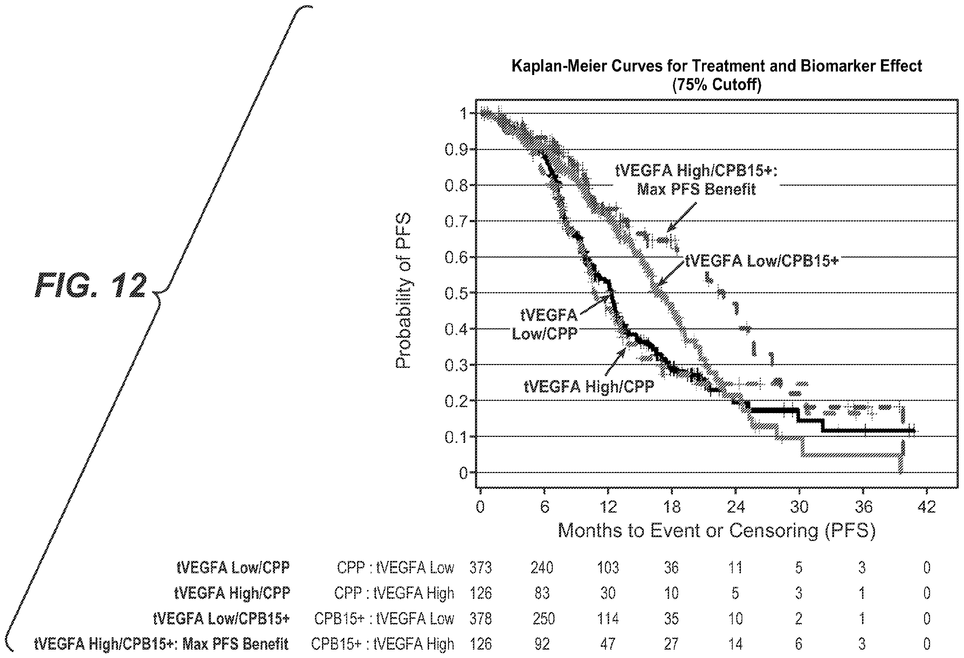

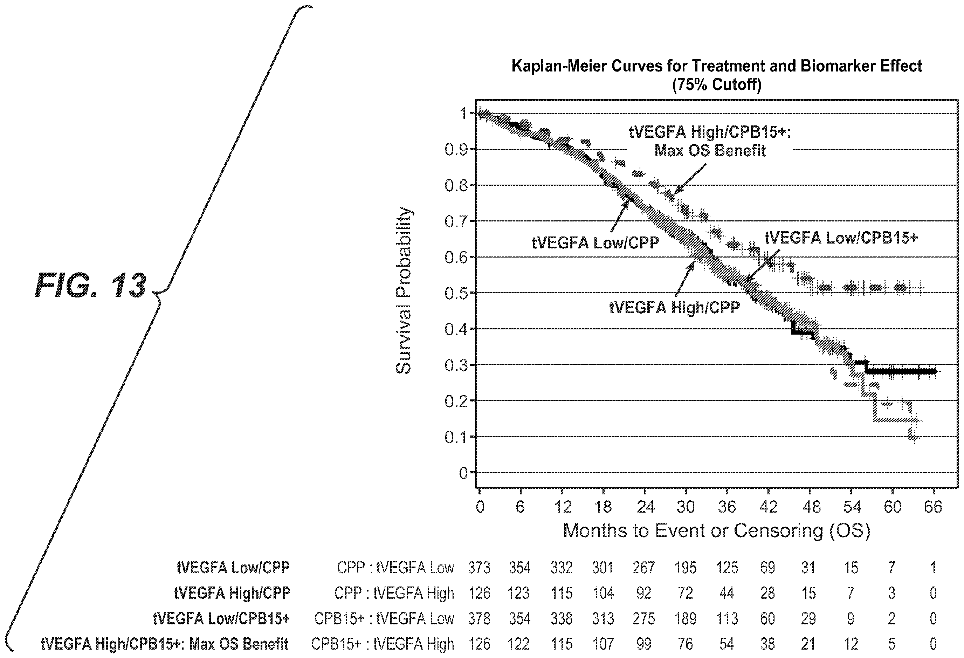

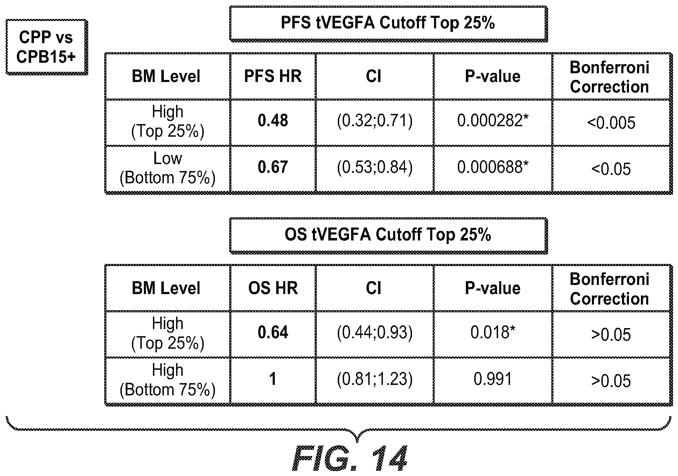

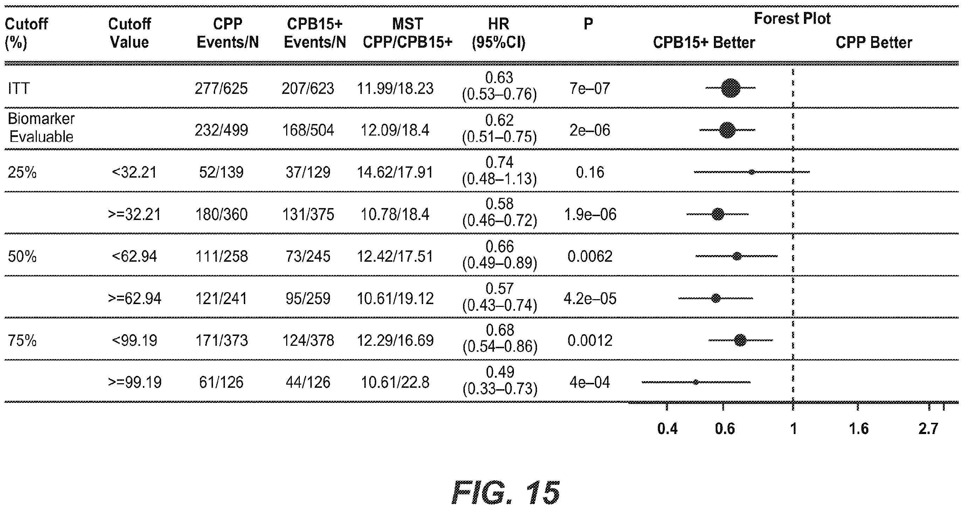

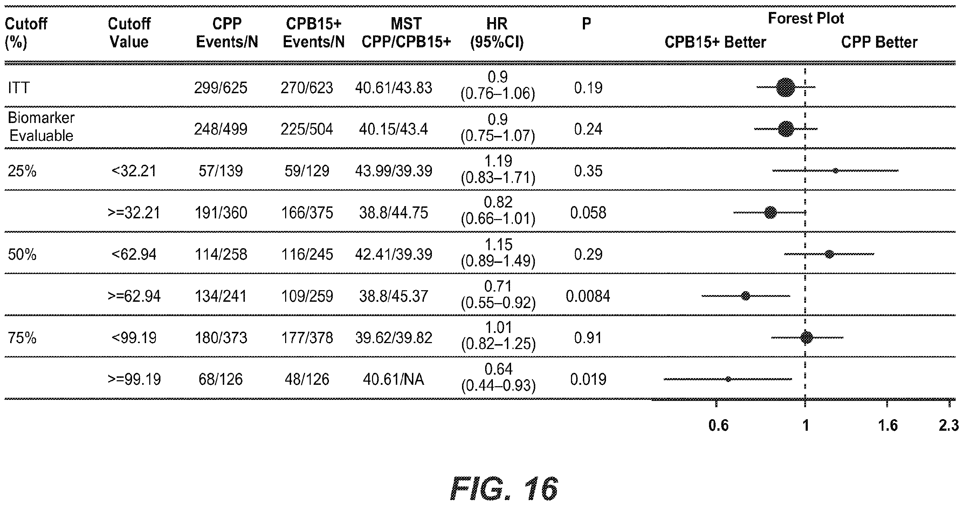

The expression of tumor VEGFA within the BEP population was further analyzed and is detailed in Figs. 12-17. In contrast to the CD31 biomarker effects, where the observed benefits were seen at a 50% cutoff (i.e., top 50% of the CD31 expression range), the benefit in PFS and OS observed with bevacizumab treatment was found at a 75% cutoff for VEGFA expression (i.e., the top 25% of the tumor VEGFA expression range). Thus, patients expressing high levels of VEGFA (i.e., above a 50% cutoff, particularly above a 75% cutoff) exhibited the maximum PFS and OS from treatment with bevacizumab (Figs. 12-14). Quartile analysis of treatment effects on PFS in patient subgroups based on % cutoff confirmed that, in general, patients with high levels of tumor VEGFA expression (i.e., above the specified % cutoff) benefited from treatment with bevacizumab (Fig. 15). Quartile analysis of treatment effects on OS in patient subgroups based on % cutoff showed that high levels of tumor VEGFA expression, particularly in the 50% and 75% cutoff resulted in a marked improvement in OS when treated with bevacizumab (Fig. 16).

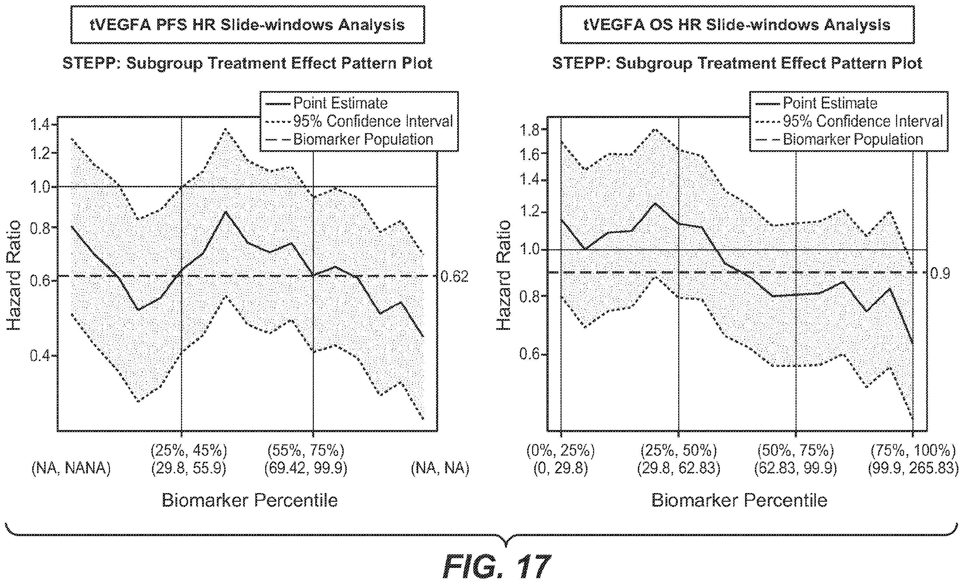

The STEPP analysis shown in Fig. 17 uses a slide window approach to demonstrate the association of tumor VEGFA expression level with hazard ratios for PFS and OS. For PFS, the hazard ratios for patients included in each subgroup represented by middle solid line were below 1.0, indicating that all patients derive some benefit from bevacizumab treatment. With respect to OS, the hazard ratio for patients above -75% of the tumor VEGFA expression range (represented by middle solid line) was below 1.0, indicating a greater benefit above -75% of the tumor VEGFA expression range (Fig. 17). Further, slide-window analysis suggested a higher optimal tumor VEGFA cutoff for PFS than for OS. Together, these results suggest that tumor VEGFA can serve as a predictive biomarker for improved PFS and OS and increased bevacizumab efficacy in ovarian cancer.

Although the foregoing invention has been described in some detail by way of illustration and example for purposes of clarity of understanding, the descriptions and examples should not be construed as limiting the scope of the invention.

## Claims

1. A VEGF antagonist for use in a method of treating a patient with ovarian cancer, the method comprising administering to the patient a therapeutically effective amount of a VEGF antagonist, wherein the VEGF antagonist is an anti-VEGF antibody and the patient's cancer has been determined to express CD31 and tumor VEGFA at a level that is more than the 75^{th} percentile for CD31 and tumor VEGFA expression, respectively, in a population of patients having the ovarian cancer.

2. The VEGF antagonist for use according to claim 1, wherein the cancer is platinum-resistant, platinum-sensitive, advanced, refractory, or recurrent.

3. The VEGF antagonist for use according to claim 1, wherein administration of the VEGF antagonist improves progression free survival (PFS) and/or overall survival (OS) in the patient.

4. The VEGF antagonist for use according to claim 1, wherein the VEGF antagonist is administered in combination with one or more additional chemotherapeutic agents in a chemotherapy regimen; optionally wherein the one or more additional chemotherapeutic agents is selected from the group consisting of: a chemotherapeutic agent, HER antibody, antibody directed against a tumor associated antigen, anti-hormonal compound, cardioprotectant, cytokine, EGFR-targeted drug, anti-angiogenic agent, tyrosine kinase inhibitor, COX inhibitor, non-steroidal anti-inflammatory drug, farnesyl transferase inhibitor, antibody that binds oncofetal protein CA 125, Her2 vaccine, HER targeting therapy, Raf or ras inhibitor, liposomal doxorubicin, topotecan, taxane, dual tyrosine kinase inhibitor, TLK286, EMD-7200, a medicament that treats nausea, a medicament that prevents or treats skin rash or standard acne therapy, a medicament that treats or prevents diarrhea, a body temperature-reducing medicament, and a hematopoietic growth factor.

5. The VEGF antagonist for use according to claim 4, wherein the chemotherapeutic agent is gemcitabine, carboplatin, oxaliplatin, irinotecan, fluoropyrimidine (e.g., 5-FU), paclitaxel (e.g., nab-paclitaxel), docetaxel, topotecan, capecitabine, leucovorin, temozolomide, interferon-alpha, or liposomal doxorubicin (e.g., pegylated liposomal doxorubicin).

6. The VEGF antagonist for use according to claim 4, wherein the chemotherapy regimen comprises administration of:
(a) carboplatin and paclitaxel; carboplatin and gemcitabine; or paclitaxel, topotecan, or pegylated liposomal doxorubicin;
(b) capecitabine and paclitaxel; or capecitabine and docetaxel;
(c) temozolomide and optionally radiotherapy;
(d) fluropyrimidine, irinotecan, cisplatin, and oxaliplatin; fluropyrimidine and irinotecan; fluropyrimidine, leucovorin, and oxaliplatin; or irinotecan, fluoropyrimidine, and leucovorin;
(e) paclitaxel and topotecan; or paclitaxel and cisplatin; and/or
(f) interferon-alpha2a.

7. The VEGF antagonist for use according to claim 1, wherein the anti-VEGF antibody is bevacizumab.

8. The VEGF antagonist for use according to claim 1, wherein CD31 and/or tumor VEGFA expression is detected by an immunohistochemical (IHC) method.

9. The VEGF antagonist for use according to claim 1, wherein the level of CD31 expression detected in said cancer of said patient is used to determine the density of CD31 microvascular structures (CD31 MVD) in said cancer of said patient, and optionally wherein CD31 MVD of said patient's cancer is compared to the 75^{th} percentile of CD31 MVD in the population of patients.

10. A method of identifying a patient suffering from ovarian cancer who may benefit from administration of a VEGF antagonist, the method comprising determining the expression level of CD31 and tumor VEGFA in a sample obtained from the patient, wherein expression of CD31 and tumor VEGFA at a level that is more than the 75^{th} percentile for CD31 and tumor VEGFA expression, respectively, in a population of patients having the ovarian cancer indicates that the patient may benefit from administration of a VEGF antagonist, wherein the VEGF antagonist is an anti-VEGF antibody.

11. The method of claim 10, wherein the cancer is platinum-resistant, platinum-sensitive, advanced, refractory, or recurrent.

12. The method of claim 10, wherein the sample is a tumor tissue sample, and/or wherein the sample is obtained before neoadjuvant or adjuvant therapy.

13. The method of claim 10, wherein CD31 and/or tumor VEGFA expression is detected by an immunohistochemical (IHC) method.

14. The method of claim 10, wherein the VEGF antagonist is formulated for administration in combination with one or more additional chemotherapeutic agents in a chemotherapy regimen, optionally wherein the one or more additional chemotherapeutic agents is selected from the group consisting of: a chemotherapeutic agent, HER antibody, antibody directed against a tumor associated antigen, anti-hormonal compound, cardioprotectant, cytokine, EGFR-targeted drug, anti-angiogenic agent, tyrosine kinase inhibitor, COX inhibitor, non-steroidal anti-inflammatory drug, farnesyl transferase inhibitor, antibody that binds oncofetal protein CA 125, Her2 vaccine, HER targeting therapy, Raf or ras inhibitor, liposomal doxorubicin, topotecan, taxane, dual tyrosine kinase inhibitor, TLK286, EMD-7200, a medicament that treats nausea, a medicament that prevents or treats skin rash or standard acne therapy, a medicament that treats or prevents diarrhea, a body temperature-reducing medicament, and a hematopoietic growth factor.

15. The method of claim 14, wherein the chemotherapy regimen comprises the administration of carboplatin and paclitaxel.

16. The method of claim 10, wherein the anti-VEGF antibody is bevacizumab.

17. The method of claim 10, wherein the level of CD31 expression detected in said sample of said patient is used to determine the density of CD31 microvascular structures (CD31 MVD) in said cancer of said patient, and optionally wherein CD31 MVD of said patient's sample is compared to the 75^{th} percentile of CD31 MVD in the population of patients.

## Patentansprüche

1. VEGF-Antagonist zur Verwendung in einem Verfahren zum Behandeln eines Patienten mit Eierstockkrebs, wobei das Verfahren das Verabreichen einer therapeutisch wirksamen Menge eines VEGF-Antagonisten an den Patienten umfasst, wobei der VEGF-Antagonist ein Anti-VEGF-Antikörper ist und bestimmt wurde, dass der Krebs des Patienten CD31 und Tumor-VEGFA auf einem Niveau exprimiert, das höher ist als die 75. Perzentile für CD31- bzw. Tumor-VEGFA-Expression in einer Population von Patienten mit dem Eierstockkrebs.

2. VEGF-Antagonist zur Verwendung nach Anspruch 1, wobei der Krebs platinresistent, platinsensitiv, fortgeschritten, refraktär oder rezidivierend ist.

3. VEGF-Antagonist zur Verwendung nach Anspruch 1, wobei die Verabreichung des VEGF-Antagonisten das progressionsfreie Überleben (PFS) und/oder das Gesamtüberleben (OS) bei dem Patienten verbessert.

4. VEGF-Antagonist zur Verwendung nach Anspruch 1, wobei der VEGF-Antagonist in Kombination mit einem oder mehreren zusätzlichen Chemotherapeutika in einem Chemotherapieregime verabreicht wird; gegebenenfalls wobei das eine oder die mehreren zusätzlichen Chemotherapeutika ausgewählt sind aus der Gruppe, bestehend aus: einem Chemotherapeutikum, HER-Antikörper, gegen ein tumorassoziiertes Antigen gerichtetem Antikörper, antihormoneller Verbindung, Kardioprotektivum, Zytokin, auf EGFR abzielendem Arzneimittel, anti-angiogenem Mittel, Tyrosinkinaseinhibitor, COX-Inhibitor, nicht-steroidalem Entzündungshemmer, Farnesyltransferaseinhibitor, Antikörper, der onkofetales Protein CA125 bindet, Her2-Impfstoff, auf HER abzielender Therapie, Raf- oder ras-Inhibitor, liposomalem Doxorubicin, Topotecan, Taxan, dualem Tyrosinkinaseinhibitor, TLK286, EMD-7200, einem Medikament, das Übelkeit behandelt, einem Medikament, das Hautausschlag vorbeugt oder behandelt oder Standard-Aknetherapie, einem Medikament, das Diarrhö behandelt oder vorbeugt, einem die Körpertemperatur senkenden Medikament und einem hämatopoetischen Wachstumsfaktor.

5. VEGF-Antagonist zur Verwendung nach Anspruch 4, wobei das Chemotherapeutikum Gemcitabin, Carboplatin, Oxaliplatin, Irinotecan, Fluoropyrimidin (z. B. 5-FU), Paclitaxel (z. B. Nab-Paclitaxel), Docetaxel, Topotecan, Capecitabin, Leucovorin, Temozolomid, Interferon-alpha oder liposomales Doxorubicin (z. B. pegyliertes liposomales Doxorubicin) ist.

6. VEGF-Antagonist zur Verwendung nach Anspruch 4, wobei das Chemotherapieregime die Verabreichung von Folgendem umfasst:
(a) Carboplatin und Paclitaxel; Carboplatin und Gemcitabin; oder Paclitaxel, Topotecan oder pegyliertes liposomales Doxorubicin;
(b) Capecitabin und Paclitaxel; oder Capecitabin und Docetaxel;
(c) Temozolomid und gegebenenfalls Strahlentherapie;
(d) Fluoropyrimidin, Irinotecan, Cisplatin und Oxaliplatin; Fluoropyrimidin und Irinotecan; Fluoropyrimidin, Leucovorin und Oxaliplatin; oder Irinotecan, Fluoropyrimidin und Leucovorin;
(e) Paclitaxel und Topotecan; oder Paclitaxel und Cisplatin; und/oder
(f) Interferon-alpha2a.

7. VEGF-Antagonist zur Verwendung nach Anspruch 1, wobei der Anti-VEGF-Antikörper Bevacizumab ist.

8. VEGF-Antagonist zur Verwendung nach Anspruch 1, wobei die CD31- und/oder Tumor-VEGFA-Expression mit einem immunhistochemischen-(IHC)-Verfahren nachgewiesen wird.

9. VEGF-Antagonist zur Verwendung nach Anspruch 1, wobei das Niveau der in dem Krebs des Patienten nachgewiesenen CD31-Expression zum Bestimmen der Dichte von CD31-mikrovaskulären Strukturen (CD31-MVD) in dem Krebs des Patienten verwendet wird, und gegebenenfalls wobei CD31-MVD des Krebses des Patienten mit der 75. Perzentile von CD31-MVD in der Population von Patienten verglichen wird.

10. Verfahren zum Identifizieren eines an Eierstockkrebs leidenden Patienten, der von der Verabreichung eines VEGF-Antagonisten profitieren kann, wobei das Verfahren das Bestimmen des Expressionsniveaus von CD31 und Tumor-VEGFA in einer von dem Patienten erhaltenen Probe umfasst, wobei die Expression von CD31 und Tumor-VEGFA auf einem Niveau, das höher ist als die 75. Perzentile für CD31- bzw. Tumor-VEGFA-Expression in einer Population von Patienten mit dem Eierstockkrebs, angibt, dass der Patient von der Verabreichung eines VEGF-Antagonisten profitieren kann, wobei der VEGF-Antagonist ein Anti-VEGF-Antikörper ist.

11. Verfahren nach Anspruch 10, wobei der Krebs platinresistent, platinsensitiv, fortgeschritten, refraktär oder rezidivierend ist.

12. Verfahren nach Anspruch 10, wobei die Probe eine Tumorgewebeprobe ist, und/oder wobei die Probe vor einer neoadjuvanten oder adjuvanten Therapie erhalten wird.

13. Verfahren nach Anspruch 10, wobei CD31- und/oder Tumor-VEGFA-Expression mit einem immunhistochemischen-(IHC)-Verfahren nachgewiesen wird.

14. Verfahren nach Anspruch 10, wobei der VEGF-Antagonist zur Verabreichung in Kombination mit einem oder mehreren zusätzlichen Chemotherapeutika in einem Chemotherapieregime formuliert wird, gegebenenfalls wobei das eine oder die mehreren zusätzlichen Chemotherapeutika ausgewählt sind aus der Gruppe, bestehend aus: einem Chemotherapeutikum, HER-Antikörper, gegen ein tumorassoziiertes Antigen gerichtetem Antikörper, antihormoneller Verbindung, Kardioprotektivum, Zytokin, auf EGFR abzielendem Arzneimittel, anti-angiogenem Mittel, Tyrosinkinaseinhibitor, COX-Inhibitor, nicht-steroidalem Entzündungshemmer, Farnesyltransferaseinhibitor, Antikörper, der onkofetales Protein CA125 bindet, Her2-Impfstoff, auf HER abzielender Therapie, Raf- oder ras-Inhibitor, liposomalem Doxorubicin, Topotecan, Taxan, dualem Tyrosinkinaseinhibitor, TLK286, EMD-7200, einem Medikament, das Übelkeit behandelt, einem Medikament, das Hautausschlag vorbeugt oder behandelt oder Standard-Aknetherapie, einem Medikament, das Diarrhö behandelt oder vorbeugt, einem die Körpertemperatur senkenden Medikament und einem hämatopoetischen Wachstumsfaktor.

15. Verfahren nach Anspruch 14, wobei das Chemotherapieregime die Verabreichung von Carboplatin und Paclitaxel umfasst.

16. Verfahren nach Anspruch 10, wobei der Anti-VEGF-Antikörper Bevacizumab ist.

17. Verfahren nach Anspruch 10, wobei das Niveau der in der Probe des Patienten nachgewiesenen CD31-Expression zum Bestimmen der Dichte von CD31-mikrovaskulären Strukturen (CD31-MVD) in dem Krebs des Patienten verwendet wird, und gegebenenfalls wobei CD31-MVD der Probe des Patienten mit der 75. Perzentile von CD31-MVD in der Population von Patienten verglichen wird.

## Revendications

1. Antagoniste du VEGF pour une utilisation dans une méthode de traitement d'une patiente atteinte d'un cancer de l'ovaire, la méthode comprenant l'administration à la patiente d'une quantité thérapeutiquement efficace d'un antagoniste du VEGF, dans lequel l'antagoniste du VEGF est un anticorps anti-VEGF et le cancer de la patiente a été déterminé pour exprimer le CD31 et le VEGFA tumoral à un niveau qui est supérieur au 75^{ème} centile pour l'expression du CD31 et du VEGFA tumoral, respectivement, dans une population de patientes atteintes d'un cancer de l'ovaire.

2. Antagoniste du VEGF pour une utilisation selon la revendication 1, dans lequel le cancer est résistant au platine, sensible au platine, avancé, réfractaire ou récidivant.

3. Antagoniste du VEGF pour une utilisation selon la revendication 1, dans lequel l'administration de l'antagoniste du VEGF améliore la survie sans progression (SSP) et/ou la survie globale (SG) chez la patiente.

4. Antagoniste du VEGF pour une utilisation selon la revendication 1, dans lequel l'antagoniste du VEGF est administré en association avec un ou plusieurs agents chimiothérapeutiques supplémentaires dans un schéma posologique de chimiothérapie ; éventuellement dans lequel le ou les agents chimiothérapeutiques supplémentaires sont choisis dans le groupe constitué par : un agent chimiothérapeutique, un anticorps anti-HER, un anticorps dirigé contre un antigène associé à une tumeur, un composé anti-hormonal, un agent cardioprotecteur, une cytokine, un médicament ciblé sur l'EGFR, un agent anti-angiogénique, un inhibiteur de la tyrosine kinase, un inhibiteur de la COX, un médicament anti-inflammatoire non stéroïdien, un inhibiteur de la farnésyl transférase, un anticorps qui se lie à la protéine oncofœtale CA 125, un vaccin anti-Her2, une thérapie ciblant HER, un inhibiteur de Raf ou de ras, la doxorubicine liposomale, le topotécan, le taxane, un double inhibiteur de la tyrosine kinase, TLK286, EMD-7200, un médicament qui traite la nausée, un médicament qui prévient ou traite une éruption cutanée ou une thérapie standard de l'acné, un médicament qui traite ou prévient la diarrhée, un médicament réduisant la température corporelle et un facteur de croissance hématopoïétique.

5. Antagoniste du VEGF pour une utilisation selon la revendication 4, dans lequel l'agent chimiothérapeutique est la gemcitabine, le carboplatine, l'oxaliplatine, l'irinotécan, la fluoropyrimidine (par exemple, le 5-FU), le paclitaxel (par exemple, le nab-paclitaxel), le docétaxel, le topotécan, la capécitabine, la leucovorine, le témozolomide, l'interféron-alpha ou la doxorubicine liposomale (par exemple, la doxorubicine liposomale pegylée).

6. Antagoniste du VEGF pour une utilisation selon la revendication 4, dans lequel le schéma posologique de chimiothérapie comprend l'administration de :
(a) carboplatine et paclitaxel ; carboplatine et gemcitabine ; ou paclitaxel, topotécan ou doxorubicine liposomale pegylée ;
(b) capécitabine et paclitaxel ; ou capécitabine et docétaxel ;
(c) témozolomide et éventuellement une radiothérapie ;
(d) fluoropyrimidine, irinotécan, cisplatine et oxaliplatine ; fluoropyrimidine et irinotécan ; fluoropyrimidine, leucovorine et oxaliplatine ; ou irinotécan, fluoropyrimidine et leucovorine ;
(e) paclitaxel et topotécan ; ou paclitaxel et cisplatine ; et/ou
(f) interféron-alpha2a.

7. Antagoniste du VEGF pour une utilisation selon la revendication 1, dans lequel l'anticorps anti-VEGF est le bévacizumab.

8. Antagoniste du VEGF pour une utilisation selon la revendication 1, dans lequel l'expression du CD31 et/ou du VEGFA tumoral est détectée par une méthode immunohistochimique (IHC).

9. Antagoniste du VEGF pour une utilisation selon la revendication 1, dans lequel le niveau d'expression du CD31 détecté dans ledit cancer de ladite patiente est utilisé pour déterminer la densité des structures microvasculaires du CD31 (CD31 MVD) dans ledit cancer de ladite patiente, et éventuellement dans lequel la CD31 MVD du cancer de ladite patiente est comparée au 75^{ème} centile de la CD31 MVD de la population de patientes.

10. Méthode d'identification d'une patiente souffrant d'un cancer de l'ovaire qui pourrait bénéficier de l'administration d'un antagoniste du VEGF, la méthode comprenant la détermination du niveau d'expression du CD31 et du VEGFA tumoral dans un échantillon de la patiente, dans laquelle l'expression du CD31 et du VEGFA tumoral à un niveau qui est supérieur au 75^{ème} centile pour l'expression du CD31 et du VEGFA tumoral, respectivement, dans une population de patientes atteintes du cancer de l'ovaire indique que la patiente pourrait bénéficier de l'administration d'un antagoniste du VEGF, dans laquelle l'antagoniste du VEGF est un anticorps anti-VEGF.

11. Méthode selon la revendication 10, dans lequel le cancer est résistant au platine, sensible au platine, avancé, réfractaire ou récidivant.

12. Méthode selon la revendication 10, dans laquelle l'échantillon est un échantillon de tissu tumoral, et/ou dans laquelle l'échantillon est obtenu avant une thérapie néoadjuvante ou adjuvante.

13. Méthode selon la revendication 10, dans laquelle l'expression du CD31 et/ou du VEGFA tumoral est détectée par une méthode immunohistochimique (IHC).

14. Méthode selon la revendication 10, dans laquelle l'antagoniste du VEGF est formulé pour une administration en association avec un ou plusieurs agents chimiothérapeutiques supplémentaires dans un schéma posologique de chimiothérapie, éventuellement dans laquelle le ou les agents chimiothérapeutiques supplémentaires sont choisis dans le groupe constitué par: un agent chimiothérapeutique, un anticorps anti-HER, un anticorps dirigé contre un antigène associé à une tumeur, un composé anti-hormonal, un agent cardioprotecteur, une cytokine, un médicament ciblé sur l'EGFR, un agent anti-angiogénique, un inhibiteur de la tyrosine kinase, un inhibiteur de la COX, un médicament anti-inflammatoire non stéroïdien, un inhibiteur de la farnésyl transférase, un anticorps qui se lie à la protéine oncofœtale CA 125, un vaccin anti-Her2, une thérapie ciblant HER, un inhibiteur de Raf ou de ras, la doxorubicine liposomale, le topotécan, le taxane, un double inhibiteur de la tyrosine kinase, TLK286, EMD-7200, un médicament qui traite la nausée, un médicament qui prévient ou traite une éruption cutanée ou une thérapie standard de l'acné, un médicament qui traite ou prévient la diarrhée, un médicament réduisant la température corporelle et un facteur de croissance hématopoïétique.

15. Méthode selon la revendication 14, dans laquelle le schéma posologique de chimiothérapie comprend l'administration de carboplatine et de paclitaxel.

16. Méthode selon la revendication 10, dans laquelle l'anticorps anti-VEGF est le bévacizumab.

17. Méthode selon la revendication 10, dans laquelle le niveau d'expression du CD31 détecté dans ledit échantillon de ladite patiente est utilisé pour déterminer la densité des structures microvasculaires du CD31 (CD31 MVD) dans ledit cancer de ladite patiente, et éventuellement dans laquelle la CD31 MVD de l'échantillon de ladite patiente est comparée au 75^{ème} centile de la CD31 MVD de la population de patientes.
